# EUROPEAN PATENT SPECIFICATION

(11) **EP 1 332 440 B1**
(45) Date of publication and mention of the grant of the patent: **11.04.2012**
(21) Application number: 01977472.8
(22) Date of filing: 04.10.2001
(51) Int. Cl.: G06F 17/00, A61M 5/00, A61M 5/142, A61M 5/172, A61B 5/00

(54) **DATA COLLECTION ASSEMBLY FOR PATIENT INFUSION SYSTEM**
ANORDNUNG ZUM ERFASSEN VON DATEN FÜR EIN INFUSIONSSYSTEM
ENSEMBLE DE COLLECTE DE DONNEES DESTINE A UN SYSTEME DE TRANSFUSION DE PATIENT

(30) Priority: 04.10.2000 US 237904 P
(43) Date of publication of application: 06.08.2003
(73) Proprietor: Insulet Corporation, Beverly, MA 01915-6120 (US)
(72) Inventor: FLAHERTY, J., Christopher, Topsfield, MA 01983 (US); GARIBOTTO, John, T., Charlestown, MA 02129 (US)
(74) Representative: Hill, Justin John
(86) International application number: PCT/US2001/031089
(87) International publication number: WO 2002/028454

(56) References cited:
- WO-A-00/10628
- WO-A-00/13580
- WO-A-00/19887
- WO-A-00/29047
- WO-A-02/20073
- US-A- 5 984 894

## Description

### Field of the Invention

The present invention relates generally to a system of medical devices, and more particularly to small, low cost, portable infusion devices that collect physiologic data from a mammalian patient, and are used to achieve precise, sophisticated, and programmable flow patterns for the delivery of therapeutic liquids to that patient.

### Background of the Invention

Today, there are numerous diseases and other physical ailments that are treated by various medicines including pharmaceuticals, nutritional formulas, biologically derived or active agents, hormonal and gene based material and other substances in both solid or liquid form. In the delivery of these medicines, it is often desirable to bypass the digestive system of a mammalian patient to avoid degradation of the active ingredients caused by the catalytic enzymes in the digestive tract and liver. Delivery of a medicine other than by way of the intestines is known as parenteral delivery. Parenteral delivery of various drugs in liquid form is often desired to enhance the effect of the substance being delivered, insuring that the unaltered medicine reaches its intended site at a significant concentration. Also, undesired side effects associated with other routes of delivery, such as systemic toxicity, can potentially be avoided.

Often, a medicine may only be available in a liquid form, or the liquid version may have desirable characteristics that cannot be achieved with solid or pill form. Delivery of liquid medicines may best be accomplished by infusing directly into the cardiovascular system via veins or arteries, into the subcutaneous tissue or directly into organs, tumors, cavities, bones or other site specific locations within the body.

Parenteral delivery of liquid medicines into the body is often accomplished by administering bolus injections using a needle and reservoir, or continuously by gravity driven dispensers or transdermal patch technologies. Bolus injections often imperfectly match the clinical needs of the patient, and usually require larger individual doses than are desired at the specific time they are given. Continuous delivery of medicine through gravity feed systems compromise the patient's mobility and lifestyle, and limit the therapy to simplistic flow rates and profiles. Transdermal patches have special requirements of the medicine being delivered, particularly as it relates to the molecular structure, and similar to gravity feed systems, the control of the drug administration is severely limited.

Ambulatory infusion pumps have been developed for delivering liquid medicaments to a patient. These infusion devices have the ability to offer sophisticated fluid delivery profiles accomplishing bolus requirements, continuous infusion and variable flow rate delivery. These infusion capabilities usually result in better efficacy of the drug and therapy and less toxicity to the patient's system. An example of a use of an ambulatory infusion pump is for the delivery of insulin for the treatment of diabetes mellitus. These pumps can deliver insulin on a continuous basal basis as well as a bolus basis as is disclosed in U.S. Patent 4,498,843 to Schneider et al.

The ambulatory pumps often work with a reservoir to contain the liquid medicine, such as a cartridge or reservoir, and use electro-mechanical pumping or metering technology to deliver the medication to the patient via tubing from the infusion device to a needle that is inserted transcutaneously, or through the skin of the patient. The devices allow control and programming via electromechanical buttons or switches located on the housing of the device, and accessed by the patient or clinician. The devices include visual feedback via text or graphic screens, such as liquid crystal displays known as LCD's, and may include alert or warning lights and audio or vibration signals and alarms. The device can be worn in a harness or pocket or strapped to the body of the patient.

Often it is desirable to modify and customize the amount or delivery profile of the liquid medication based on specific physiologic parameters that are measurable with state of the art diagnostic devices. For the diabetic patient, blood glucose levels can be measured with a glucose monitoring device such as the One Touch device, manufactured by LifeScan a division of Johnson and Johnson. Based on the blood glucose readings measured, the patient or clinician can give specific amounts of liquid insulin or modify the flow profile of the insulin currently being delivered to the patient. For other diseases and therapies, physiologic variables such as blood pressure, white blood cell count, toxicity level and various other parameters may be measured to determine the best amount, rate and timing of liquid medication to be delivered to the patient, easily modified by those patients utilizing an ambulatory infusion device.

Currently available ambulatory infusion devices are expensive, difficult to program and prepare for infusion, and tend to be bulky, heavy and very fragile. Filling these devices can be difficult and require the patient to carry both the intended medication as well as filling accessories. The devices require specialized care, maintenance, and cleaning to assure proper functionality and safety for their intended long term use. Due to the high cost of existing devices, healthcare providers limit the patient populations approved to use the devices and therapies for which the devices can be used.
WO 02/20073 (EP1335764) discloses a device for delivery of a fluid to a patent and a remote control device. WO 00/10628 discloses an infusion system for infusing a liquid into a body and includes an external infusion device and a remote commander WO 00/29047 discloses a device for supplying medicaments to a patient which can be wirelessly programmed. WO 00/19887 discloses a system comprising a sensor for producing a signal indicative of a characteristic of a user, a transmitter and a remotely located data receiving device.

Also, the currently available ambulatory infusion devices are not specifically designed to work or be compatible with diagnostic devices, requiring additional equipment, supplies and most importantly programming steps for each infusion device to fully take advantage of modifying the flow profile based on the diagnostic measurement.

Clearly, therefore, there was a need for a programmable and adjustable infusion system that is precise and reliable and can offer clinicians and patients a small, low cost, light weight, simple to use alternative for parenteral delivery of liquid medicines.

In response, the applicant of the present application provided a small, low cost, lightweight, easy to use device for delivering liquid medicines to a patient, which is described in copending U.S. application US 2002 /072733 A1 filed on August 31, 2001. The device includes an exit port, a dispenser for causing fluid from a reservoir to flow to the exit port, a local processor programmed to cause a flow of fluid to the exit port based on flow instructions from a separate, remote control device, and a wireless receiver connected to the local processor for receiving the flow instructions. To reduce the size, complexity and costs of the device, the device is provided with a housing that is free of user input components, such as a keypad, for providing flaw instructions to the local processor.

None of the above disclose devices for delivering fluid to a patient as disclosed herein. Preferably, the fluid delivery devices will be simple in design, and inexpensive and easy to manufacture, to further reduce the size, complexity and costs of the devices, such that the devices or portions thereof lend themselves to being small and disposable in nature. In addition, the fluid delivery devices will preferably be compatible with a diagnostic device.

### Summary of the Invention

The applicant has determined that a sophisticated ambulatory infusion device that can be programmed to reliably deliver variable flow profiles of liquid medications, yet is small, lightweight and low cost, is needed. Avoiding the general upkeep and maintenance required by expensive, long-term use devices is necessary for broader acceptance of ambulatory infusion therapy. Smaller and lighter devices are easier to carry and are more comfortable for the patient even allowing the device to attach with adhesive to the patient's skin similar to a transdermal patch.

An inexpensive device allows greater flexibility in prescribing the device for use by reducing the financial burden on healthcare insurance providers, hospitals and patient care centers as well as patients themselves. In addition, low cost devices make it more practical for a patient to have one or more replacement devices readily available. If the primary device is lost or becomes dysfunctional, availability of the replacement eliminates costly expedited repair and avoids periods of discontinued ambulatory therapy.

The present invention, therefore, provides a system as defined in claim 1 for low cost infusion of liquid medications into the body of a mammalian patient while monitoring one or more of the patient's physiologic parameters. In accordance with the present invention, a small, light weight and low cost fluid delivery device capable of adjustable and programmable fluid delivery includes a housing that surrounds a reservoir chamber. In fluid communication with the reservoir chamber is a dispenser for dispensing the fluid from the reservoir in finite amounts. The dispenser is controlled by an electronic microcontroller (referred to as the "local processor") of the fluid delivery device. The fluid delivery device further includes a communication element that receives information from a remote control device not mechanically attached to the fluid delivery device of the present invention. Also included is an exit port assembly in fluid communication with the dispenser from which the liquid medication exits the fluid delivery device and enters the body of a mammalian patient transcutaneously.

The types of liquids that could be delivered by the fluid delivery device of the present invention include but are not limited to: insulin, antibiotics, nutritional fluids, total parenteral nutrition or TPN, analgesics, morphine, hormones or hormonal drugs, gene therapy drugs, anticoagulants, analgesics, cardiovascular medications, AZT or chemotherapeutics. The types of medical conditions that the fluid delivery device of the present invention might be used to treat are diabetes, cardiovascular disease, pain, chronic pain, cancer, AIDS, neurological diseases, Alzheimer's Disease, ALS, Hepatitis, Parkinson's Disease or spasticity.

The housing of the fluid delivery device is free of electromechanical elements, such as switches or buttons, that the patient would press to program or alter the programming of the fluid delivery device. The primary interface between the fluid delivery device and the user is via the remote control device.

The system further includes a data collection assembly, which can be a separate device or integrated into either the fluid delivery device or the remote control device. The data collection assembly collects data from a sensor. The sensor may be implanted under the skin of the patient, located on the skin of the patient, or work with a sample, such as blood, that has been taken from the patient and brought near or in contact to the sensor.

These aspects of the invention together with additional features and advantages thereof may best be understood by reference to the following detailed descriptions and examples taken in connection with the accompanying illustrated drawings.

### Brief Description of the Drawings

Fig. 1a is a sectional view of an example of a fluid delivery device;

Fig. 1b is a perspective view of an embodiment of a remote control device constructed in accordance with the present invention for use with the fluid delivery device of the invention;

Fig. 1c is a perspective view of an embodiment of a data collection assembly constructed in accordance with the present invention for use with the fluid delivery device of the invention and the remote control device of Fig. 1b to form a system according to the present invention;

Fig. 2 is a sectional view of an example of a fluid delivery device;

Fig. 2a is an enlarged sectional view of a dispenser of the fluid delivery device of Fig. 2 shown with an empty accumulator;

Fig. 2b is an enlarged sectional view of the dispenser of the fluid delivery device of Fig. 2 shown with the accumulator filled;

Fig. 3 is a sectional view of an example of a fluid delivery device;

Fig. 4 is a sectional view of an other example of a fluid delivery device;

Fig. 5 is a sectional view of another embodiment of the remote control device constructed in accordance with the present invention;

Fig. 6 is a sectional view of another example of a fluid delivery device;

Fig. 7 shows another embodiment of the system constructed in accordance with the present invention and including a remote control device shown in perspective view, and a fluid delivery device and a data collection assembly shown in a diagrammatic view affixed to a patient;

Fig. 8 shows an additional embodiment of the system constructed in accordance with the present invention and including a remote control device and a data collection assembly shown in perspective view, and a fluid delivery device shown in a diagrammatic view affixed to a patient;

Fig. 9 is a sectional view of an embodiment of the fluid delivery device constructed in accordance with the present invention;

Fig. 10 is a sectional view of a further embodiment of the fluid delivery device constructed in accordance with the present invention;

Fig. 11 is a sectional view of an additional embodiment of the remote control device constructed in accordance with the present invention;

Fig. 12a is a sectional view of an additional embodiment of the fluid delivery device constructed in accordance with the present invention

Fig. 12b is a top plan view of the fluid delivery device of Fig. 12a;

Fig. 13 is a sectional view of a further embodiment of the fluid delivery device constructed in accordance with the present invention;

Fig. 14 is a top plan view of an embodiment of a shipping package constructed in accordance with the present invention and shown containing an embodiment of the fluid delivery device;

Fig. 14a is a sectional view of the shipping package taken along line 14a-41a of Fig. 14, and wherein the fluid delivery device is shown partially cut-away;

Fig. 14b is a perspective view of the remote control device of the system of the present invention;

Fig. 14c is a perspective view of the data collection assembly of the system of the present invention;

Fig. 14d is a top view of an insulin cartridge provided as part of the system of the present invention; and

Fig. 14e is a top view of a sterile infusion set provided as part of the system of the present invention.

Like reference characters designate identical or corresponding components and units throughout the several views.

### Detailed Description of the Preferred Embodiments

Set forth herebelow are detailed descriptions of certain embodiments and examples of the fluid delivery systems, devices, and kits as well as methods of the present invention.

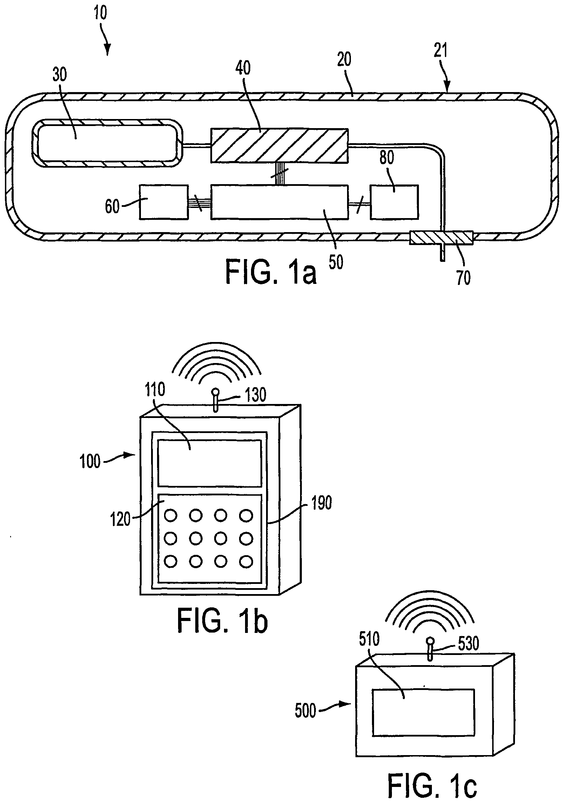

In Fig. 1a, there is illustrated, generally at 10, a fluid delivery device. The fluid delivery device 10 includes a housing 20 that surrounds numerous internal components including a reservoir 30. The reservoir 30 has a collapsible design such as a metal bellows or is made of a collapsible material such as a silicone elastomer. The volume of reservoir 30 is chosen to best suit the therapeutic application of the fluid delivery device 10 impacted by such factors as available concentrations of medicinal fluids to be delivered, acceptable times between refills or disposal of the fluid delivery device 10, size constraints and other factors. For treatment of Type I diabetic patients, a reservoir of less than 5 ml, specifically 3 ml is appropriate. The reservoir 30 is in fluid communication with a dispenser 40.

An electronic microcontroller (referred to as "local processor") 50 controls the activation of the dispenser 40. The electronic microcontroller 50 contains all the programming information and electronic circuitry and memory needed to allow the user to program the desired flow patterns and adjust the programming as necessary. Such circuitry can include microprocessors, digital and analog integrated circuits, resistors, capacitors, transistors and other semiconductors and other electronic components known to those skilled in the art. The electronic microcontroller 50 also includes programming, electronic circuitry and memory to properly activate the dispenser at the needed time intervals. A power supply 80, such as a battery or capacitor, may be included to supply power to the electronic microcontroller 50.

An exit port assembly 70 is in fluid communication with the dispenser 40. When the electronic microcontroller 50 activates the dispenser 40, a specific amount of fluid exits the fluid delivery device 10 via the exit port assembly 70. The exit port assembly can include elements to penetrate the skin of the patient, or can connect to a standard infusion device that includes transcutaneous delivery means.

The housing 20 preferably is free of any electromechanical switches or buttons on its surface or otherwise accessible to the user to adjust the programming included in the electronic microcontroller 50. In order to program or adjust the programming of the electronic microcontroller 50, the fluid delivery device 10 includes a communication element 60 which can receive signals from a separate device.

In Fig. 1b, a remote control device 100 is shown which can communicate with the fluid delivery device 10 of Fig. 1a via a communication element 60 of the device 10. Signals are sent via the controller communication element (not viewable in Fig. 1b), which may be connected to an antenna 130 shown as being external to the device 100.

The remote control device 100 includes user interface components including an array of electromechanical switches, such as the membrane keypad 120 shown. Also included is a visual display 110 such as a liquid crystal display or LCD. A touch screen can alternatively be provided. Although not shown, the remote control device 100 also includes its own electronic microcontroller (referred to as "remote processor") connecting the user interface components to the controller communication element.

The patient or clinician can program the fluid delivery device 10 by entering information into the remote control device 100 which can download information from the controller communication element 160 to the communication element 60 with each key stroke or button pressed or in a batch mode of multiple key strokes. Complex flow algorithms, requests for bolus delivery and other desired infusions of the medicinal fluid can be accomplished by entering information into the remote control device 100 which is then transmitted to the fluid delivery device 10. The communication can be confirmed as acceptable by the fluid delivery device 10 electronic microcontroller 50 by using one ore more features such as standard handshaking protocols, redundant transmissions and other communication confirmation methods as are known to those skilled in the art.

The lack of electromechanical switches results in a reduction in the cost of the device and greatly reduces the size and surface area requirements. It also allows the housing outer surface 21 to be relatively smooth simplifying cleaning and preventing items such as sweaters from catching on edges. Since the remote control device 100 also includes a visual display 110, the fluid delivery device 10 can be void of an information screen, further reducing cost, size and weight. Lacking electromechanical switches and information screens greatly simplifies the design of the fluid delivery device 10 to be made more flexible and resistant to damage.

The remote control device 100 may include various personal data assistant (PDA) functions such as calendar and date books, address functions, e-mail handling, and games such as Trophy Bass 4 manufactured by Sierra Sports. Alternatively, the remote control device 100 may include the entire electronics and user interface to function as a cellular telephone. Integration with or into such commercial devices as PDA's or Cellular telephones may have a strong appeal to patients, potentially reducing the number of handheld devices that are carried in their daily lives, or at least making the handheld remote control device 100, multi-functional and more practical.

Shown in Fig. 1c is a data collection assembly 500 provided in accordance with the present invention for use with the devices 10, 100 of Figs. 1a and 1b. In the embodiment shown, the data collection assembly 500 includes a user interface such as visual display 510. The data collection assembly 500 may communicate with either the fluid delivery device 10 or the remote control device 100, or the data collection assembly 500 may communicated with both the fluid delivery device 10 and the remote control device 100, independently. Such information transfer can be accomplished with wireless electronic communication, or by electromechanically attaching the data collection assembly 500 to either device. Such electromechanical attachment can consist of a male plug on one device and a female receptacle on the other. The data collection assembly 500 may include antenna 530, shown in Fig. 1c as external, to facilitate the wireless communication such as radio frequency or RF communication signals.

In a preferred embodiment, the data collection assembly 500 is integrated into the fluid delivery device 10 or the remote control device 100. The data collection assembly 500 may collect data from a sensor that has been implanted under the skin, a sensor that is attached on or near the body of the patient, or a separate sensor that analyzes a samples removed from the patient, such as a blood sample. Alternatively, the data collection assembly 500 may include an integrated sensor, and directly determine the physiologic parameter, or perform an analysis on a biologic sample from the patient.

In another preferred embodiment, the data collection assembly 500 is integrated into either the fluid delivery device 10 or the remote control device 100 and is designed to communicate with a separate diagnostic device such as a glucometer or blood analysis machine. Information is transferred from the separate diagnostic device to the data collection assembly 500 via a direct electronic connection or via wireless communication as are described above. The information is stored in the data collection assembly 500, and may be made available to the user, may be used to assist in modifying the current or future programming of the device 10, or may directly modify the programming creating a partial or full closed loop fluid delivery system.

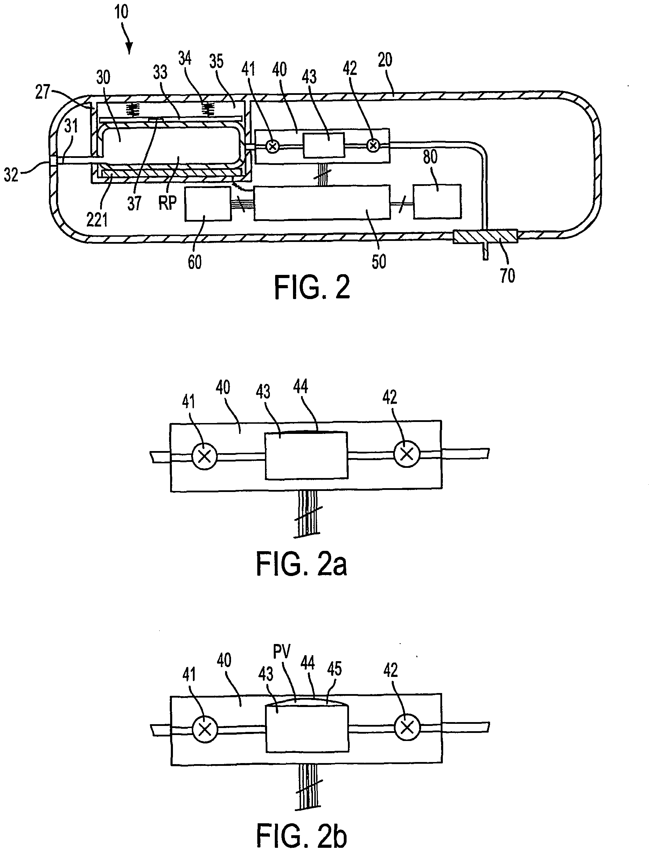

Fig. 2 shows an example of the fluid delivery device 10
where the reservoir 30 is made of a flexible material and is enclosed in the reservoir chamber 35 defined by the housing 20 and housing reservoir walls 27. The reservoir 30 is placed in compression by compressing member 33 attached to one end of compressing springs 34 which are affixed at their other end to the housing 20 causing the fluid inside the reservoir 30 to be at a pressure above atmospheric pressure. In a preferred embodiment, the cross sectional area of the compressing member 33 approximates the cross sectional area of the reservoir 30. Alternatively, the housing 20 may include a flexible cantilever beam that contacts the reservoir 30 creating a pressure within reservoir 30 above atmospheric pressure. The housing 20 may include holes or slits, not shown, to perform as vents, maintaining the contents of the reservoir at or near room temperature.

In another alternative, the reservoir chamber 35 may be sealed to prevent any leaking, and filled with a gas or vapor plus fluid mixture surrounding the reservoir 30 to place the fluid within the reservoir under pressure above atmospheric pressure. The gas can be air, or the vapor plus fluid mixture could be Freon. The Freon vapor plus fluid mixture provides the design advantage of near constant pressure if the fluid delivery device 10 is maintained at near constant temperature. In an alternative embodiment, the amount of gas placed in a sealed reservoir chamber 35 may be chosen such that the reservoir 30 pressure is less than atmospheric for the entire full to empty conditions of the reservoir 30.

The reservoir 30 may be prefilled by the device manufacturer or a cooperating drug manufacturer, or may include external filling means consisting of a fill assembly 31. If the fluid delivery device 10 is prefilled by the manufacturer, the memory of the electronic module 50 can contain various information regarding the prefilled drug including but not limited to type or name, concentration and volume. The fill assembly can include a needle insertion septum 32. The reservoir 30 and other fluid path components may be placed in a vacuum during the final manufacturing process to simplify filling and priming of the fluid delivery device 10 for the patient. Needle insertion septum 32 may be constructed of a resealing elastomer such as silicone and allow a needle to puncture through to add fluid to the reservoir 30. An alternative to the needle insertion septum 32 is a standard fluid connection, such as a Luer connector, which can be affixed to the fill assembly 31 in combination with a one way valve such as a duck bill valve, not shown. The patient could attach a syringe filled with the liquid medication to the Luer connector and fill the fluid delivery device 10. The fill assembly 31 may be designed so that the patient can fill the fluid delivery device 10 one time only, such as by having the Luer connection break off when syringe is removed.

The dispenser 40 is in fluid attachment with the reservoir 30. The dispenser may include an inlet valve 41, and outlet valve 42, and an accumulator 43 therebetween. Since the fluid is maintained at a pressure above atmospheric pressure, opening of the inlet valve 41 allows the accumulator to fill to the reservoir pressure, after which the inlet valve is 41 is closed. At the proper time determined by the electronic microcontroller 50 programming, the outlet valve 42 can be opened, dispensing fluid to the exit port assembly 70, which is at the pressure of the patient, or atmospheric pressure. The accumulator will then be at atmospheric pressure, and the outlet valve 42 can be closed, ready for another repeat cycle. The exit port assembly 70 can include a needle for transcutaneous placement or a standard Luer assembly for attachment to a transcutaneous needle set.

The dispenser 40 of the device of Fig. 2 does not create a driving or pumping force on the fluid passing therethrough, but rather acts as a metering device, allowing pulses of fluid to pass through the dispenser 40 from a pressure in reservoir 30 above atmospheric, to pressure at the exit port assembly 70 equal to atmospheric. The dispensing inlet valve 41 and outlet valve 42 of the dispenser 40 is controlled by the electronic microcontroller 50. The electronic microcontroller 50 includes the electronic programming, controls and circuitry to allow sophisticated fluid delivery programming and control of the dispenser 40.

Fig 2a shows the dispenser 40 where the accumulator 43 is at atmospheric pressure. An accumulator membrane 44 is shown in its non-distended state, caused by atmospheric pressure only. Inlet valve 41 is closed, and outlet valve 42 may be open or closed, but must have been opened since the last time inlet valve 41 was opened.

Fig 2b shows the condition where outlet valve 42 was closed, and inlet valve 41 had been opened. Because of the elevated pressure of the fluid from reservoir 30, the accumulator membrane 44 is distended thus increasing the volume of accumulator 43 by an accumulator volume 45. After inlet valve 41 is closed, outlet valve 42 can be opened, dispensing accumulator volume 45 and allowing accumulator 42 to retract to the position shown in Fig 2a. The inlet valve 41 and outlet valve 42 of the dispenser 40 and the electronic microcontroller 50 are designed to prevent both valves from ever being open at the same time, precluding the reservoir 30 to ever flow directly to the exit port assembly 70. The prevention of both valves being open at the same time is critical and can be accomplished via mechanical means, electrical means or both. The prevention can be accomplished in the dispenser 40 design, the electronic microcontroller 50 design, or both.

The dispenser 40 shown in Figs. 2, 2a and 2b dispense finite pulses of fluid volume, the pulse volume PV, with each series of activations. The pulse volume PV is determined by the properties, materials and construction of accumulator 43 and its accumulator membrane 44. Pulse volumes delivered by infusion devices are typically chosen to be small relative to what would be considered a clinically significant volume. For insulin applications at a concentration of 100 units per ml, less than a 2 microliter pulse, typically 1 microliter, is appropriate. If the fluid delivery device 10 were to be programmed via the remote control device 100 to deliver 2 units an hour, the dispenser would deliver 20 pulses an hour, or a pulse every 3 minutes. Such pulsitile flow is continued continuous if the pulse size is small enough. Other drugs or concentrations permit a much larger pulse size. Various flow rates are achieved by adjusting the time between pulses. To give a fixed volume or bolus, multiple pulses are given in rapid succession until the bolus volume is reached.

The pulse volume PV may not always be constant enough to be within the accuracy requirements of the fluid delivery device 10. One factor impacting pulse volume PV is reservoir pressure. The fluid delivery device 10 may include means of monitoring reservoir pressure RP and adjust the timing between pulses to achieve the desire flow pattern. An example of such compensation would be to decrease time between pulses as pulse volume PV decreases to maintain the programmed flow rate. Means of monitoring such parameters as reservoir pressure RP are described below. Alternative to monitoring pressure would be to monitor the volume of reservoir 30. Each time a pulse or series of pulses were delivered, the feedback could determine if a proper amount had been delivered, both for individual pulses and cumulative intended volume to have been infused, compensating as errors were detected. Such volume transducer means is also described below.

The electronic microcontroller is attached to a communication element 60 which receives electronic communication from the remote control device 100 using radio frequency or other wireless communication standards and protocols. The information transferred includes codes or packets of codes that the electronic microcontroller 50 uses to confirm that the information was received correctly, similar to the way standard telephone modem communication is performed. More sophisticated codes can be included to allow the information to be self-corrected or pinpoint the area of bad information. In a preferred embodiment, the communication element 60 is a two-way communication element allowing the fluid delivery device to send information back to the remote control device 100. In that particular embodiment, the remote control device 100 integral controller communication element 160 is a receiver as well as a transmitter allowing it to receive the information sent back by the fluid delivery device 10.

Also included in the fluid delivery device 10 of Fig. 2 is a power supply 80 for delivering the energy needed by the microcontroller 50. The power supply 80 may be integrated into the fluid delivery device 10 and not accessible to the user. In an alternative embodiment, the user may insert the power supply 80, typically a battery, into the device. In another embodiment, the power supply 80 may consist of an integrated battery or capacitor, for minimal power requiring devices, such as the electronic memory, and a user inserted battery for powering the remainder of the electronic microcontroller 50. Other components that may require electrical energy are the communication element 60, the dispenser 40 and other components such as sensors or transducers. Fig. 2 includes a reservoir transducer 37, such as a volume transducer such as that described in U.S. Patent 5,533,389 to Kamen et al. Fig. 2 also includes a pressure transducer 221, located on the housing reservoir walls 27 and in contact with a portion of the reservoir 30. The pressure transducer 221 may consist of force sensing resistor technology such as that manufactured by Interlink, Inc. of Camarillo, CA. Reservoir transducer 37 or pressure transducer 221 can transmit information to electronic microcontroller 50 to indicate how and when to activate the dispenser 40 or other parameter determining flow as well as conditions such as the reservoir being empty, loss of pressure or leak, under or over infusion, etc.

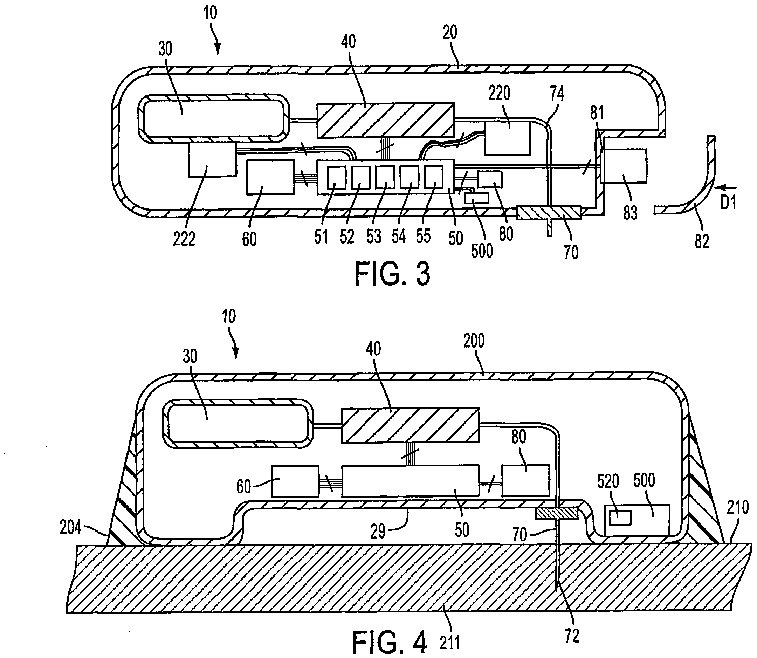

Fig. 3 depicts another example of the fluid delivery device 10 including sensors providing feedback to the electronic microcontroller 50, an electronic assembly for the various electronic devices and an optional second power supply, potentially a battery and insertable by the user by opening a battery door. Included is a housing 20 surrounding a reservoir 30 which is prefilled during the manufacturing process or alternatively can be filled by the user as is described above. The reservoir 30 is in fluid connection with dispenser 40. The fluid in reservoir 30 may not be under pressure, or may be at a pressure below atmospheric pressure requiring dispenser 40 to include a mechanism to pump the fluid from reservoir 30. Such pumping means may be a peristaltic drive as is familiar to those skilled in the art. If the fluid of reservoir 30 is at a pressure above atmospheric, dispenser 40 may consist of a fluid metering device without pumping capability as is described above. The dispenser 40 is attached to exit port assembly 70 through which fluid exits the fluid delivery device 10. The dispenser 40 is activated by electronic microcontroller 50 that receives electrical energy from power supply 80. The programming of the electronic microcontroller 50 is adjusted by information received via communication element 60 from a remote control device (similar to the device 100 of Fig. 1b).

As shown in Fig 3, fluid delivery device 10 may have various sensors which feedback information to the electronic microcontroller 50. The reservoir 30 may have in its proximity a volume sensor 222, as is described above, whose signals are interpreted by the electronic microcontroller 50. Alternatively, a pressure sensor, also described above, could be in contact with reservoir 30. Also shown is an occlusion sensor 220 downstream of dispenser 40 and in approximation to exit port tubing lumen 74. Other types of sensors which may be integrated include but are not limited to an occlusion detector, a reservoir volume transducer, a reservoir empty detector, a leak detector, a voltage monitor, a photodetector, a pressure transducer, a fluid contact detector, an impedance monitor or a vibration monitor

The electronic microcontroller 50, may include a microprocessor 51, memory 52, an electronic clock oscillator 53, an analog to digital converter 54 and a multiplexer 55. Also shown in Fig. 3 is an optional secondary power source 83, attached by the user to battery connector 81, and providing electrical power to the electronic microcontroller 50. A battery door 82 is removed for insertion and then reattached by sliding in direction D1 to the housing 20 of fluid delivery device 10. In a preferred embodiment, power supply 80 provides electrical power for memory retention and low power electronics only, and secondary power source 83 provides electrical power for higher consumption devices such as the dispenser 40. Both power supply 80 and secondary power source 83 may be consumer batteries, such as alkaline or nickel cadmium batteries, or other energy storage devices such as a capacitor. Additionally, both power supply 80 and secondary power source 83 may be rechargeable power sources.

Also shown in Fig. 3 is an example in which the data collection assembly 500 is integrated into the fluid delivery device 10. The data collection assembly 500, which is preferably electrically connected to electronic microcontroller 50, may be used to collect or store information related to a physiologic parameter of the patient. The data collection assembly 500 may include an integrated sensor, not shown, or a communication element, also not shown. The communication element may be used to communicate with a separate diagnostic device such as a glucometer. In one embodiment, the data collection assembly may consist of a combination of the communication element 60 of the fluid delivery device 10 and the electronic microcontroller 50. The communication element 60 can communicate with a separate diagnostic device using wireless communication, similar to the communication with the remote control device 100, and the information can be stored in the memory of the electronic microcontroller.

The information that is collected by the data collection assembly 500 may be used to feed back to the patient, via the fluid delivery device 10 or remote control device 100, signify an alarm condition, potentially activating a audio or tactile alarm, assist in programming the device with user participation, or automatically modify the programming of the device, potentially alerting the user of the change.

Fig. 4 depicts another example of the device wherein the fluid delivery device 10 includes means of attaching the device to the skin of the patient. The fluid delivery device 10 includes an integrated data collection assembly 500 that further comprises a DCA sensor assembly 520. The device includes a recessed housing 200 that includes a housing recessed surface 29. The recessed housing 200 surrounds a reservoir 30 in fluid communication with dispenser 40. The reservoir can be filled with the medicinal fluid during the manufacturing process or can include means of the patient or caregiver filling the reservoir, not shown. The fluid in reservoir 30 may not be under pressure, or may be at a pressure below atmospheric pressure requiring dispenser 40 to include a mechanism to pump the fluid from reservoir 30. Such pumping means may be a peristaltic drive as is familiar to those skilled in the art. If the fluid of reservoir 30 is at a pressure above atmospheric, dispenser 40 may consist of a fluid metering device without pumping capability as is described above. The dispenser 40 is attached to exit port assembly 70, which terminates in skin penetrating cannula 72.

The skin penetrating cannula 72 can be a rigid member such as a needle, or a flexible cannula. The skin penetrating cannula 72 is inserted through the skin prior to attaching the fluid delivery device to the skin and may be inserted by a needle insertion assistance device, often spring loaded, and known to those skilled in the art. Such a spring loaded mechanism may be integrated into the fluid delivery device 10, not shown. Fig. 4 depicts the skin penetrating cannula 72 transcutaneously entering the patient through the surface of patient's skin 210 and entering subcutaneous tissue 211.

The dispenser 40 is activated by electronic microcontroller 50 at specific intervals specified by its programming to achieve the desired flow volume or rate. The electronic microcontroller receives electrical energy from power supply 80. The programming of electronic microcontroller 50 is adjusted by information received via communication element 60 from a remote control device (similar to the device 100 of Fig. 1b).

The data collection assembly 500 may be located near the portion of the housing 20 which is placed in contact with the surface of the patient's skin 210. The DCA sensor assembly 520 includes means of measuring a physiologic parameter. The physiologic parameter can be blood glucose level measured with reflected light or other known technologies, temperature measured with a thermocouple or other known technologies, pressure measured with a transducer or other known technologies, a parameter of blood measured with a needle and vacuum removal assembly, all not shown, or other physiologic parameter that may be valuable in relation to the fluid delivery therapy.

When attaching the fluid delivery device 10 to the patient's skin, the data collection assembly 500 and DCA sensor assembly 520 may be positioned near a previously implanted sensor to facilitate proper analysis, reading or communication with said sensor. Alternatively, the data collection assembly 500 may include the sensor itself, with the sensor being positioned near, at or below the skin when the fluid delivery device is attached to the skin of the patient. The sensor may be positioned far from the transcutaneous entry site of the skin penetrating cannula 72 of exit port assembly 70, as far as can be permitted by the cross sectional area of the fluid delivery device 10, in order to avoid potential unwanted impact of direct fluid delivery affecting the physiologic parameter.

Alternatively, the sensor may be attached directly to the skin penetrating cannula 72, attachment not shown, and inserted under the skin simultaneous with the skin penetrating cannula 72 being inserted under the skin. The DCA sensor assembly 520 is designed to work in conjunction with said sensor, and would be placed in proximity to the transcutaneous cannula/sensor pair, also not shown.

Alternatively, the data collection assembly 500 may include a communication element 540, not shown, to communicate with a separate diagnostic device to collect physiologic data.

Fig. 4 also includes adhesive axial projections 204, which are attached to the fluid delivery device 10 and are used to affix the fluid delivery device 10 to the surface of the patient's skin 210. Depicted in Fig. 4 are two projections forming a single axis, and connected to the side of the fluid delivery device 10. Alternatively, four projections, one from each side of a top view square shaped fluid delivery device 10 may be included, or a continuous piece of adhesive, square cut, that covers the entire device with a boundary significantly larger than the boundary of fluid delivery device 10 to fixedly attach fluid delivery device 10 to the surface of the patient's skin 210, not shown. The adhesive used on the adhesive axial projections 204, is such that the fluid delivery device will remain attached to the patient for the duration of use, typically 2-4 days, and be removed. The adhesive axial projections 204 may be connected to the side of fluid delivery device 10, the top, the bottom, or any combination of surfaces as long as the adhesive side projecting out from the fluid delivery device 10 is facing downward as it related to the orientation the fluid delivery device is intended to be placed when attached to the patient. Typically 2 to 4 projections will be provided.

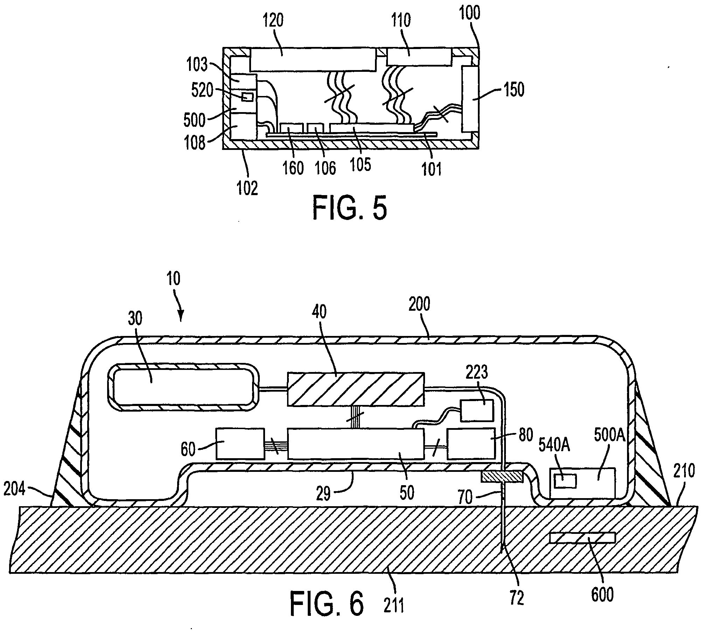

Figs. 5 and 6 depict another preferred embodiment of the system wherein the remote control device 100 includes the data collection assembly 500. The fluid delivery device may include a second data collection assembly 500A. The remote control device may include a remote control device alarm transducer 103 and the fluid delivery device may include an alarm transducer 223. The device includes a recessed housing 200 that includes a housing recessed surface 29. The recessed housing 200 surrounds a reservoir 30 in fluid communication with dispenser 40. The reservoir can be filled with the medicinal fluid during the manufacturing process or can include means of the patient or caregiver filling the reservoir, not shown. The fluid in reservoir 30 may not be under pressure, or may be at a pressure below atmospheric pressure requiring dispenser 40 to include a mechanism to pump the fluid from reservoir 30. Such pumping means may be a peristaltic drive as is familiar to those skilled in the art. If the fluid of reservoir 30 is at a pressure above atmospheric, dispenser 40 may consist of a fluid metering device without pumping capability as is described above. The dispenser 40 is attached to exit port assembly 70 which terminates in skin penetrating cannula 72. The skin penetrating cannula 72 can be a rigid member such as a needle, or a flexible cannula. The skin penetrating cannula 72 is inserted through the skin prior to attaching the fluid delivery device to the skin and may be inserted by a needle insertion assistance device, often spring loaded, and known to those skilled in the art. Such a spring loaded mechanism may be integrated into the fluid delivery device 10, not shown. Fig. 5 depicts the skin penetrating cannula 72 transcutaneously entering the patient through the surface of patient's skin 210 and entering subcutaneous tissue 211.

The dispenser 40 is activated by electronic microcontroller 50 at specific intervals specified by its programming to achieve the desired flow volume or rate. The electronic microcontroller receives electrical energy from power supply 80. The electronic microcontroller 50 programming is adjusted by information received via communication element 60 from a remote control device 100.

The fluid delivery device 10 of Fig. 6 also includes adhesive axial projections 204 which are attached to the fluid delivery device 10 and are used to affix the fluid delivery device 10 to the surface of the patient's skin 210. Depicted in Fig. 6 are two projections forming a single axis, and connected to the side of the fluid delivery device 10. Alternatively, four projections, one from each side of a top view square shaped fluid delivery device 10 may be included, or a continuous piece of adhesive, square cut, that covers the entire device with a boundary significantly larger than the boundary of fluid delivery device 10 to fixedly attach fluid delivery device 10 to the surface of the patient's skin 210, not shown. The adhesive used on the adhesive axial projections 204, is such that the fluid delivery device will remain attached to the patient for the duration of use, typically 2-4 days, and be removed. The adhesive axial projections 204 may be connected to the side of fluid delivery device 10, the top, the bottom, or any combination of surfaces as long as the adhesive side projecting out from the fluid delivery device 10 is facing downward as it related to the orientation the fluid delivery device is intended to be placed when attached to the patient. Typically 2 to 4 projections will be provided.

In Fig. 6, the data collection assembly 500 is integral to the remote control device 100. The data collection assembly 500 includes a DCA sensor assembly 520 which is used to measure a physiologic parameter. Alternatively, the data collection assembly may include a DCA sensor communication element 540, not shown, to communicate with a separate device used to measure a physiologic parameter. The information collected by the data collection assembly 500 is transferred to the internal programming of the remote control device 100 and may be additionally transferred to the memory of the fluid delivery device 10 via wireless communication described above. The information collected can be made available to the patient or clinician, used to assist in programming of the fluid delivery device 10, used to determine or modify an alarm condition or to activate an alarm transducer, or the information can be used to automatically modify, with or without user confirmation, the future fluid delivery profile.

An optional second data collection assembly 500A may be included in the system, shown in Fig. 6 as integral to the fluid delivery device. The second data collection assembly 500A may include a second DCA sensor communication element 540A for communicating with non-integrated sensor assembly 600, shown in Fig. 6 implanted in the subcutaneous tissue under the patient's skin, or DCA sensor communication element 540A may communicate with a separate diagnostic device. Alternatively or additionally, the second data collection assembly 500A may include a second DCA sensor assembly 520A, not shown, for directly or indirectly measuring a physiologic parameter.

Since the functions of the data collection assembly 500 and second data collection assembly 500A are the same, and a third data collection assembly could be included, it is implied that having a separate data collection assembly device, and integrating into either or both of the fluid delivery device 10 and remote control device 100 are all embodiments within the scope of this application.

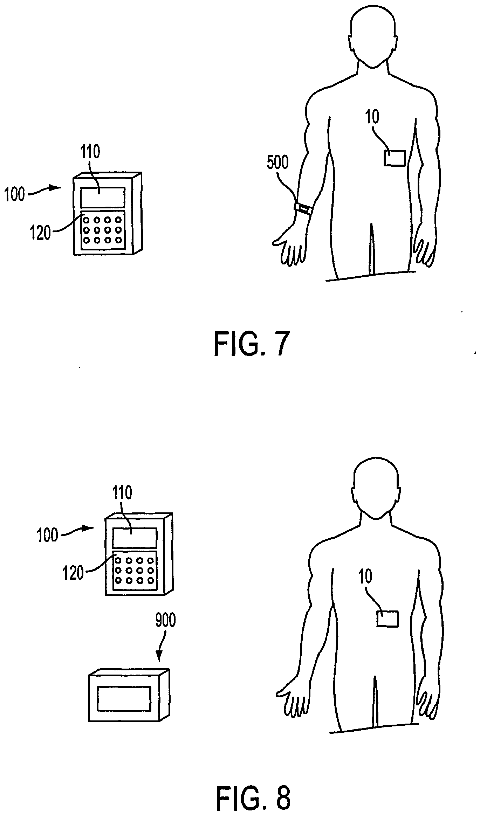

Fig. 7 depicts a preferred embodiment of the present invention defining a system including a remote control device 100, a fluid delivery device 10 that is affixed to the patient 800, preferably in the abdominal area, and a data collection assembly 500 that is attached to the wrist of patient 800. The data collection assembly 500 may communicate with either or both the fluid delivery device and the remote control device 100. In a typical application, the data collection assembly 500 may include glucose sensing technology, such as that developed by Cygnus Corporation of California, and may communicate with either device via electromechanical connection, as described above, or via wireless communication, also described above.

Fig 8 depicts another preferred embodiment of the present invention defining a system including a remote control device 100, a fluid delivery device 10 that is affixed to the patient 800, preferably in the abdominal area, and a separate diagnostic device 900. At least one of the fluid delivery device 10 or the remote control device 100 will include a data collection assembly 500, not shown, which will receive information from the separate diagnostic device 900. The communication between devices with be accomplished via electromechanical connection, as described above, or via wireless communication, also described above.

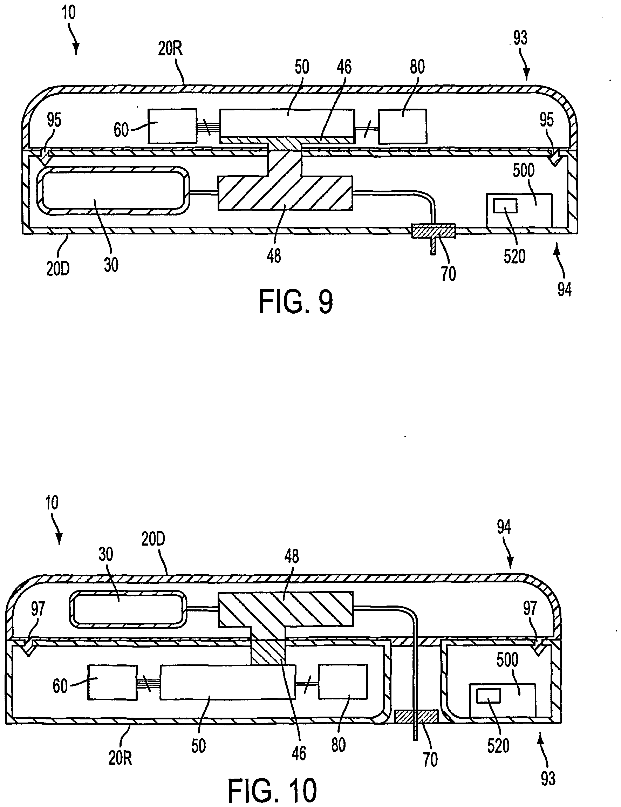

Fig. 9 depicts an embodiment of the fluid delivery device 10 of the invention, which comprises a reusable assembly 93 and a disposable assembly 94 such that when the two assemblies are connected, the exit port assembly 70 exits from the disposable assembly 94 in a direction away from the reusable assembly 93. The fluid delivery device includes a data collection assembly 500 for collection of physiologic data. The disposable assembly 94 includes a housing 20D that surrounds a reservoir 30 in fluid communication with a fluid metering element 48. The reservoir 30 can be filled with the medicinal fluid during the manufacturing process or can include means of the patient or caregiver filling the reservoir, not shown. The fluid in reservoir 30 may not be under pressure, or may be at a pressure below atmospheric pressure requiring fluid metering element 48 to include a mechanism to pump the fluid from reservoir 30. Such pumping means may be a peristaltic drive as is familiar to those skilled in the art. If the fluid of reservoir 30 is at a pressure above atmospheric, fluid metering means 48 may consist of a fluid metering device without pumping capability as is described above. The fluid metering means 48 is attached to exit port assembly 70. Exit port assembly 70 may terminate in a standard Luer connection, not shown, and be connected to a standard transcutaneous infusion set, also not shown.

The reusable assembly 93 includes a housing 20R that surrounds a communication element 60, electronic microcontroller 50, metering control means 46 and power supply 80. The metering control means 46 is activated by electronic microcontroller 50 at specific intervals specified by its programming. Metering control means 46 activates fluid metering element 48 to achieve the desired flow volume or rate. The electronic microcontroller receives electrical energy from power supply 80. The electronic microcontroller 50 programming is adjusted by information received via communication element 60 from a remote control device (similar to the device 100 of Fig. 1b).

The reusable assembly 93 and disposable assembly 94 can be connected by the user utilizing reusable assembly snaps 95 which are received by mating holes or cutouts in the disposable assembly, to mechanically attach the two assemblies. Alternatively, the snaps may be present on the disposable assembly 94. Alternative means of attachment, not shown, include mating threads, adhesive bonds, Velcro, and other attachment means. In all configurations, both attachment and separation of the two assemblies is preferred. Multiple disposable assemblies 94 may be attached and removed from a single reusable assembly 93.

As shown in Fig. 9, the data collection assembly 500 contained in the disposable assembly 94 further comprises a DCA sensor assembly 520 which is used to measure a physiologic parameter. Alternatively, the data collection assembly may include a DCA sensor communication element 540, not shown, to communicate with a separate device used to measure a physiologic parameter. The information collected by the data collection assembly 500 is transferred to the internal programming of the fluid delivery device 10 and may be additionally transferred to the memory of a remote control device (similar to the device 100 of Fig. 1b) via wireless communication described above. The information collected can be made available to the patient or clinician, used to assist in programming of the fluid delivery device 10, used to determine or modify an alarm condition or to activate an alarm transducer, or the information can be used to automatically modify, with or without user confirmation, the future fluid delivery profile.

Fig. 10 depicts another embodiment of the fluid delivery device 10 including a reusable assembly 93 and a disposable assembly 94. When the two assemblies 93, 94 are connected, the exit port assembly 70 exits from the disposable assembly 94 in a direction toward and through the reusable assembly 93. The fluid delivery device also includes a data collection assembly 500 for collection of physiologic data. The disposable assembly 94 includes a housing 20D that surrounds a reservoir 30 in fluid communication with a fluid metering element 48. The reservoir 30 can be filled with the medicinal fluid during the manufacturing process or can include means of the patient or caregiver filling the reservoir, not shown. The fluid in reservoir 30 may not be under pressure, or may be at a pressure below atmospheric pressure requiring fluid metering element 48 to include a mechanism to pump the fluid from reservoir 30. Such pumping means may be a peristaltic drive as is familiar to those skilled in the art. If the fluid of reservoir 30 is at a pressure above atmospheric, fluid metering means 48 may consist of a fluid metering device without pumping capability as is described above. The fluid metering means 48 is attached to exit port assembly 70. Exit port assembly 70 may terminate in a standard Luer connection, not shown, and be connected to a standard transcutaneous infusion set, also not shown.

The reusable assembly 93 includes a housing 20R which surrounds a communication element 60, electronic microcontroller 50, metering control means 46 and power supply 80. The metering control means 46 is activated by electronic microcontroller 50 at specific intervals specified by its programming. Metering control means 46 activates fluid metering element 48 to achieve the desired flow volume or rate. The electronic microcontroller receives electrical energy from power supply 80. The electronic microcontroller 50 programming is adjusted by information received via communication element 60 from a remote control device (similar to the device 100 of Fig. 1b).

The reusable assembly 93 and disposable assembly 94 can be connected by the user utilizing disposable assembly snaps 97 which are received by mating holes or cutouts in the reusable assembly, to mechanically attach the two assemblies. Alternatively, the snaps may be present on the reusable assembly 93. Alternative means of attachment, not shown, include mating threads, adhesive bonds, Velcro, and other attachment means. In all configurations, both attachment and separation of the two assemblies is preferred. Multiple disposable assemblies 94 may be attached and removed from a single reusable assembly 93.

The data collection assembly 500 contained the reusable assembly 93 further comprises a DCA sensor assembly 520 which is used to measure a physiologic parameter. Alternatively or additionally, the data collection assembly may include a DCA sensor communication element 540, not shown, to communicate with a separate device used to measure a physiologic parameter. The information collected by the data collection assembly 500 is transferred to the internal programming of the fluid delivery device 10 and may be additionally transferred to the memory of the remote control device (similar to device 100 of Fig. 1b) via wireless communication described above. The information collected can be made available to the patient or clinician, used to assist in programming of the fluid delivery device 10, used to determine or modify an alarm condition or to activate an alarm transducer, or the information can be used to automatically modify, with or without user confirmation, the future fluid delivery profile.

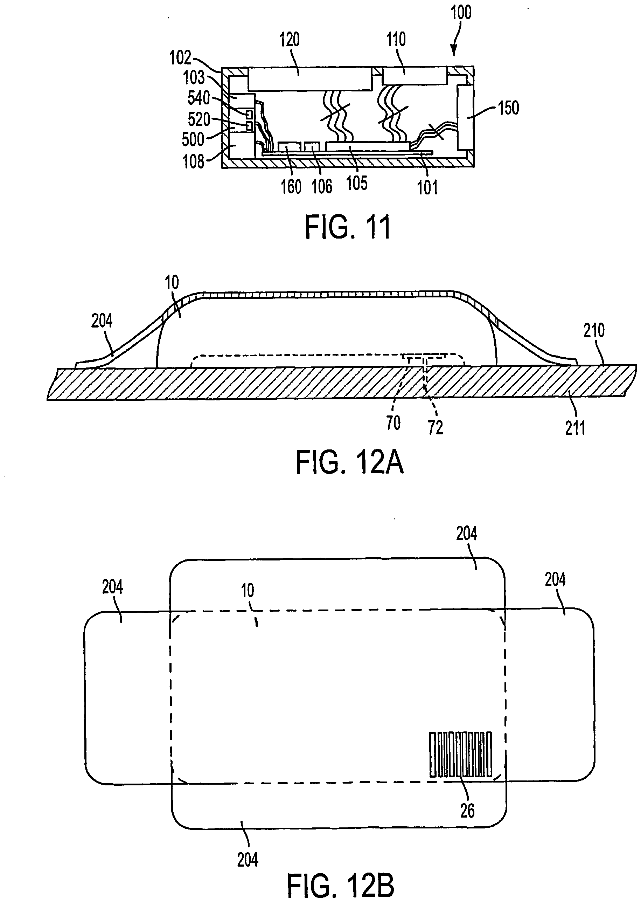

Fig. 11 depicts another preferred embodiment of the remote control device 100 of the present invention including a data collection assembly 500 which has integral to it either or both a DCA sensor 520 assembly or a DCA sensor communication element 540. The remote control device 100 further comprises a remote control device alarm transducer 103. The remote control device 100 is a hand held device that includes a controller housing 102, on which is mounted a visual display 110, such as a liquid crystal display or LCD. The visual display 110 can visually indicate status of programming, amounts, timing, and other parameters of medicinal fluid delivery. Other information can include time of day, address book, to do lists and calendar information and potentially an entertainment interface such as computerized bass fishing or other popular hand held computer game. Another use of the visual display 110 is to display information received or to be sent to devices other than fluid delivery device such as a glucometer used by diabetic patients or other diagnostic device, especially those whose information is related to the desired infusion rates and volumes to be delivered by fluid delivery device. The remote control device 100 may have a diagnostic device, such as a blood glucose monitor or glucometer, or an implantable glucose sensor reader, integrated into it, simplifying the requirements of the patient by not having to carry and maintain two separate devices. Other diagnostic devices include but are not limited to blood diagnostic devices, electrocardiography devices and readers, electroencephalogram or EEG devices and readers, blood pressure monitors and pulse oxymetry devices. Alternative to full integration of the diagnostic device, would be connection to the device via wireless or hardwired communication means, to perform a transfer of information.

The visual display 110 can also include information such as warning and alarm conditions based on the status of the fluid delivery device. Elements such as indicator lights, buzzers, and vibrational alarms may also be included in the remote control device 100 as alternative or redundant means of communicating information to the user.

The user can get information and adjust the programming of the device by depressing various electromechancal switches also mounted on controller housing 102. These switches may be joined in a bank of switches and included in membrane keypad 120 as shown in Fig. 11 and as is common with hand held electronic devices. It is preferred that the choice of electromechanical switches of the membrane keypad 120 interface with the visual display 110 in a menu driven fashion making reading information and programming the device more user friendly for the user. In an alternative embodiment, the visual display 110 and membrane keypad 120 can be combined into a single device such as a touch screen display, also common to electronic devices. Combination of touch screen displays, membrane keypads and singular switches may all be integrated into the remote control device (similar to the device 100 of Fig. 1b).

The remote control device 100 may include various electromechanical jacks, which can accept electromechanical plugs from various devices. In the embodiment of Fig. 11, a glucometer port 150 is provided. Additional connections may include ports for a bar code reader or a computer. These ports can allow two way transfer of information to enhance the capabilities of remote control device 100 and improve user friendliness. The membrane keypad 120, the visual display 110 and the port 150 are attached to the controller electronics 105. Other ports would also be attached to the controller electronics. The controller electronics are mounted and soldered to the controller printed circuit board 101 as is the controller communication element 160.

The controller communication element 160 is designed to transmit information to the communication element 60 of the fluid delivery device 10. In a preferred embodiment, both the communication element 60 and the controller communication element 160 are two way communication assemblies allowing two way communication between the remote control device 100 and fluid delivery device 10. In order to send wireless information the communication element 60 and the controller communication element 160 may include inductive wire loops or other transmitting antenna means. Information can be sent using amplitude or frequency modulation, and can be broadcast in the radio frequency, or RF range. Standard information confirmation techniques such as handshaking or checksum protocols can be used to insure accurate information transfer. With two-way communication, when errors are detected, the transfer can be repeated until acceptable, a similar technique to that utilized with two way pager technology commonplace today.

If the fluid delivery device 10 is prefilled prior to patient use, the electronic memory of electronic microcontroller 50 may contain information regarding the fluid including but not limited to type or name, concentration, amount, volume, additional drugs in solution and any diluting agents. This information can be transmitted from the fluid delivery device 10 via its communication element 60, and uploaded into the remote control device 100 via its controller communication element 160. Other information may be factory installed into the fluid delivery device 10 including but not limited to manufacturing date, expiration date, sterilization date, therapy information such as defined flow profiles and even patient or hospital information. This information can be uploaded into the remote control device 100 as described above, and the remote control device 100 may adjust its internal programming based on the information received.

In a preferred embodiment, the electronic memory of the fluid delivery device 10 includes the latest program of the remote control device 100 available at the time of manufacture of the fluid delivery device 10. Similarly, the electronic memory of the remote control device 100 includes the latest program of the fluid delivery device 10, available at the time of manufacture of the remote control device 100. At the first communication between the remote control device 100 and the fluid delivery device 10, a program check is performed, and if a newer software version for either device is available from the other device, and the existing hardware is compatible, another feature which can be programmed into both devices, the newer program is downloaded into memory and used by the upgraded device. The embedded program may be contained in read only memory, or ROM, while the downloaded program can be written into electronically writeable memory. The automatic update feature, available for each device to upgrade the other, is another way to make sure the user has the best available product for use.

Another advantageous feature associated with two-way communication is the addition of a proximity alarm. The design of the fluid delivery device 10 and remote control device 100 electronics can be such that when the distance between the two devices is greater than a particular radial length, one or both of the devices will alert the user, potentially with an audio alarm. The alarming distance should be chosen so that it is less than the communication range of the two devices. A method of creating the alarm is for the fluid delivery device 10 to send out frequent packets of information at a predetermined rate and at an amplitude or power less than the normal communication power, providing a safety margin for the proximity detection. The remote control device 100 programming expects to receive this communication at the predetermined rate, and lack of receipt of one or more of these packets, causes the remote control device 100 to activate its audio alarm 106. Alternatively or additionally, a vibrational alarm may be included. Proximity alarms may be included that do not require two way communication, by integrating a device such as a magnet into the housing 20 of fluid delivery device 10, and integrating magnetic field detection means into the remote control device 100. When the remote control device 100 magnetic field detection means do not detect the presence of fluid delivery device 10 magnetic field, the remote control device 100 activates controller audio alarm 106.

Still referring to Fig. 11, the remote control device 100 includes a controller power supply 108 that powers the various electronic components including the controller electronics 105, controller audio alarm 106. The controller power supply 108 may be a standard battery and in the preferred embodiment, the power supply 108 may be replaceable by the user by removing a battery door, not shown, and replacing after power supply 108 is inserted and attached. In an alternative embodiment, the power supply is integrated into the remote control device 100, and can be recharged with a separate device or contains enough power to supply the device for its intended length of use.

The data collection assembly 500 contained the remote control device 100 further comprises a DCA sensor assembly 520 which is used to measure a physiologic parameter. Alternatively or additionally, the data collection assembly may include a DCA sensor communication element 540, to communicate with a separate device used to measure a physiologic parameter. The information collected by the data collection assembly 500 is transferred to the internal programming of the remote control device 100 and may be additionally transferred to the memory of the fluid delivery device 10 via wireless communication described above. The information collected can be made available to the patient or clinician, used to assist in programming of the fluid delivery device 10, used to determine or modify an alarm condition or to activate an alarm transducer, or the information can be used to automatically modify, with or without user confirmation, the future fluid delivery profile.

The remote control device alarm transducer can be an audio alarm, such as a beeper, or a vibrational alarm such as a rotating eccentric shaft. The remote control device alarm transducer can be activated when various alarm conditions are encountered, such as those present in the either the fluid delivery device 10 or the remote control device 100. The alarm condition may be determined based on information collected by the integral data collection assembly 500. The remote control device 100 can also be designed to function not only as a programming device for fluid delivery device 10, but also as a key chain for carrying the patient's personal keys such as house or car keys.

Fig. 12a is a sectional side view, taken at an end side view of fluid delivery device 10. The fluid delivery device 10 includes adhesive axial projections 204, from each of four sides of fluid delivery device 10. The adhesive axial projections 204 are used to affix the fluid delivery device 10 to the surface of patient's skin 210. Alternatively, a square or circular shaped boundary adhesive material could be used, not shown, to affix to the surface of the patient's skin 210. The adhesive axial projections 204 are shown attached to the top surface of fluid delivery device 10, it should be appreciated by those skilled in the art, that the adhesive projections 204 could be attached to the side or bottom of fluid delivery device 10 and could be in various geometric configurations, shapes, sizes and lengths.

Fig. 12a devices exit port assembly 70 comprising skin penetrating cannula 72 which penetrates the surface of patient's skin 210 and enters subcutaneous tissue 211. The connection from the exit port assembly 70 to the skin penetrating cannula 72 can be a permanent connection made by the manufacturer or can be a user connectable assembly. For the purposes of cost reduction, transcutaneous penetration means that are connected by the manufacturer may be appropriate.

Fig. 12b is a top view of the fluid delivery device 10 prior to attachment to the patient. Shown are four adhesive axial projections 204, and information barcode 26 which can contain various pieces of information pertaining to fluid delivery device 10, such as manufacturing date, pre-filled therapeutic fluid information, expiration date, clinician or patient information, and other pre-determined facts. This information can be read by a separate device such as the remote control device (similar to the device 100 of Fig. 1b).

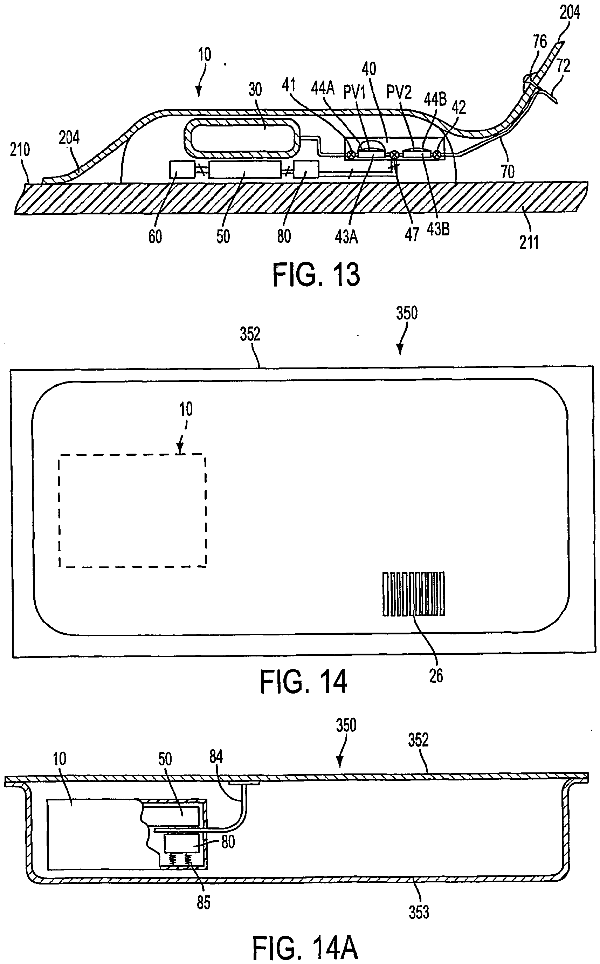

Fig. 13 shows another preferred embodiment of the fluid delivery device 10 of the present invention. In this embodiment, the exit port assembly 70 is integrated into an adhesive axial projection 204. In addition, in this preferred embodiment, the dispenser 40 consists of multiple liquid accumulators, first accumulator 43A and second accumulator 43B which are utilized to improve device performance.

The fluid delivery device 10 includes adhesive axial projections 204, projecting from two or more of the sides of fluid delivery device 10. The adhesive axial projections 204 are used to affix the fluid delivery device 10 to the surface of patient's skin 210. Alternatively, a square or circular shaped boundary adhesive material could be used, not shown, to affix to the surface of the patient's skin 210. The adhesive axial projections 204 are shown attached to the top surface of fluid delivery device 10, it should be appreciated by those skilled in the art, that the adhesive projections 204 could be attached to the side or bottom of fluid delivery device 10 and could be in various geometric configurations, shapes, sizes and lengths.

The sectional side view of fluid delivery device 10 shown in Fig. 13 depicts the exit port assembly 70 attached to one of the adhesive axial projections 204, such that the action of penetrating the surface of patient's skin 210 can be accomplished at the same time as affixing the appropriate adhesive axial projection 204 to the patient. The exit port assembly 70 further comprises a cannula access septum 76, which can be accessed with a penetrating member such as a needle or stylet, not shown. The cannula access septum 76 is designed to seal around the penetrating member during access, and repeatedly reseal after access and removal preventing leakage. The needle or stylet in combination with the cannula access septum 76 can be used to assist in the initial skin penetration step after which the needle or stylet is removed, or to achieve subsequent fluid access to the skin penetrating cannula 72. The exit port assembly 70 and skin penetrating cannula 72 are preferably preattached by the manufacturer.

Also depicted in the side cross-sectional view of fluid delivery device 10 of Fig. 13, is a dispenser 40 which comprises two accumulator assemblies, first accumulator 43A which is designed to accumulate a fixed volume of fluid PV1 when first accumulator membrane 44A is fully expanded to the limits of the cavity of first accumulator 43A, which occurs at a broad range of reservoir, or input pressures, and a second accumulator 43B which is designed to accumulate a fixed volume of fluid PV2 when second accumulator membrane 44B is fully expanded to the limits of the cavity of second accumulator 44B, which occurs at a broad range of input pressures. The volumes, PV1 and PV2 may be chosen such that PV1 is greater than PV2 and potentially a fixed multiple of PV2 volume, volumes that are determined by the size of each cavity for both first accumulator 43A and second accumulator 44B. Assuming that each accumulator has a fixed volumetric error, such that the percentage error of first accumulator 43A is less than the percentage error of second accumulator 43B, the activation of both accumulators can be made such as to reduce overall error of the system. In a practical application, PV1 can be 10 microliters at 0.5 microliter accuracy, or 5% potential error. PV2 can be 1 microliters at 0.5 microliter accuracy, or 50% potential error.

In use, inlet valve 41 can be opened to fill first accumulator 43A by expanding the first membrane 44A to contact the cavity walls of first accumulator 43A driven by the pressurized fluid from reservoir 30. After a predetermined fill time, inlet valve 41 is closed. Intermediate valve 47 must remain closed during the filling of the first accumulator 43A. Second accumulator 43B is filled by opening intermediate valve 47 while maintaining outlet valve 42 in a closed state, expanding second membrane 44B to contact the cavity walls of second accumulator 43B driven by the pressure of the fluid in first accumulator 43A. Intermediate valve 47 remains open for the second accumulator 43B predetermined fill time after which intermediate valve 47 is closed. Therapeutic fluid is delivered to the patient via exit port assembly 70 when outlet valve 42 is opened for an appropriate time to allow second accumulator membrane 44B to contract, expelling second accumulator pulse volume PV2. Note that any time outlet valve 42 is open, both intermediate valve 47 and inlet valve 41 must be closed. In the example, after 10 open and close cycles of outlet valve 42, delivering 10 pulses of volume PV2, no more that 10.0 microliters plus or minus 0.5 microliters will have been delivered based on the 5% accuracy of first accumulator 43A. This multiple accumulator design prevents over infusion inaccuracy to ever be greater than the accuracy of the first accumulator 43A for an amount of volume equal to PV1.

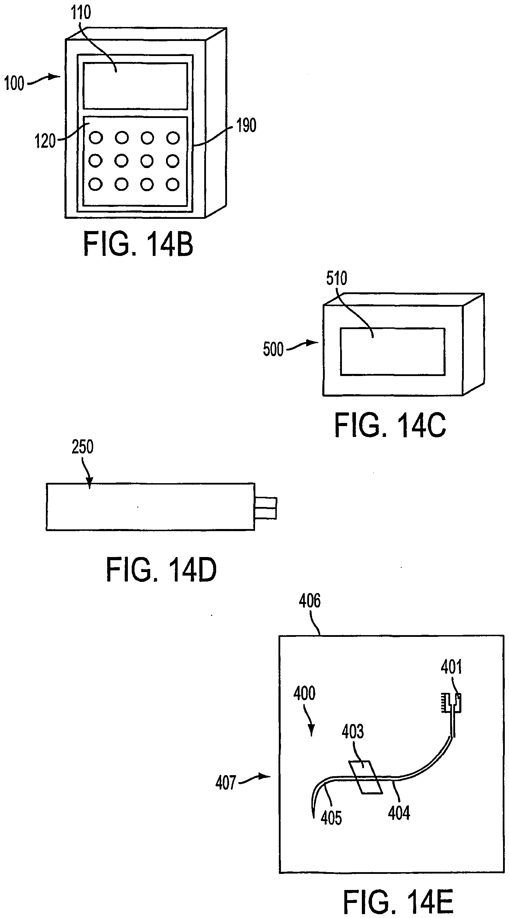

Fig. 14 depicts the fluid delivery device 10 packaged in a container. Described in Fig. 14a is means of automatically activating the power supply of the fluid delivery device 10. The fluid delivery device 10 is contained within a sterile assembly pack 350 including a sterile assembly lid 352 which may be made of Tyvek material manufactured by Dupont.

An information barcode 26 may be included on the sterile assembly pack 350, preferably on the sterile assembly lid 352. The information barcode 26 can include various pieces of information regarding the status of that particular fluid delivery device 10 such as type, volume and concentration of drug prefilled in the device, expiration date of device or drug, manufacture date of device or drug, serial numbers, lot numbers, hospital name, clinician name, patient name, prescription requirements and various other pieces of information. The barcode information can be read into a hospital or home computer, or in the preferred embodiment is uploaded via a barcode reader integral to the remote control device 100.

The fluid delivery device 10 and remote control device 100 electronics and programming can be designed such that the bar code must be read prior to programming or otherwise using the fluid delivery device 10. This feature can greatly reduce programming errors such as those associated with the patient entering drug information. If the patient were to enter a drug concentration that was incorrect, and did all the remaining programming in units of drug, instead of volume, which is common practice, while the device would function properly, all of the volumes delivered would be inaccurate based on the ratio of the incorrect concentration entered versus the true concentration of the drug being delivered. Many drugs are available in multiple concentrations such as insulin often made available to patients in 40, 50 and 100 units per ml concentrations.

The fluid delivery device 10 may be packaged individually or with various other kit components, such as a transcutaneous infusion set if not integral to the fluid delivery device 10. Alternatively, a portion of the fluid delivery device surrounding the exit port assembly 70 may be covered, sealed and sterilized with a sterility maintaining covering such as Tyvek, not shown, avoiding the need for a tray and the sterile assembly lid 352.

Fig. 14a is a cross sectional side view at the edge of fluid delivery device 10. The sterile assembly lid 352 is sealed to the sterile assembly tray 353 forming a microbial barrier such that when the sterile assembly 350 is sterilized, the fluid delivery device contained within the sterile assembly 350 remains sterile. The sterile assembly tray 353 may be made of various material types, such as PETG, and of sufficient thickness and construction to protect the fluid delivery device during shipment and storage, and may include geometries to stabilize the fluid delivery device 10 thus preventing movement. Alternatively, the sterile assembly tray 353 may be a flexible bag, also sealed with the sterile assembly lid 352 to create a sterile container.

The sterile assembly pack 350 further comprises a FDD activation tether 84 which is fixedly attached at one end to sterile assembly lid 352. The other end of FDD activation tether 84 is located between power supply 80 and electronic microcontroller 50, such that when constructed of an electrically insulating material, prevents flow of electrons from the power supply to the electronic microcontroller. When the sterile assembly lid 352 is removed, the FDD activation tether 84 is pulled out from between the power supply 80 and the electronic microcontroller 50. The power supply 80 may be spring biased with one or more battery springs 85 such that when the FDD activation tether 84 is removed, the power supply 80 makes electrical connection with the electronic microcontroller 50.

It should be appreciated by those skilled in the art, that various other activation means and methods can be accomplished with the FDD activation tether 80. The FDD activation tether 80 could be attached to the tray, causing the above actuation when the fluid delivery device 10 is removed from the sterile assembly tray 353. Alternatively, the FDD activation tether 84 could activate a switch within the fluid delivery device 10, not shown, could magnetically make an electrical connection, could remove a separate insulative material, could remove a covering thus allowing air to contact a portion of the power supply needed for activation, or various other means, all not shown. Automatic activation of the pump may increase life of the device, such as battery life, improve safety, simplify use and correlate opening the package with the chronological start of use.

The fluid delivery device 10 of the system of the present invention may be sold to hospitals, pharmacies, outpatient centers or the patients themselves. If the fluid delivery device is intended for short term or disposable use, it may be practical to sell each device with various accessories or groups of accessories that are convenient for the user. It may be desirable for certain parts of the fluid delivery device, or accessories such as an attachable transcutaneous infusion set, such as that described hereabove, to be packaged sterilized in a protective packaging. Proper aseptic maintenance of the portion of the skin that receives the transcutaneous access is important to prevent infection. Figs. 14b, 14c, 14d and 14e depict various components that may be packaged together in kit form.

Fig. 14b depicts the remote control device 100 of the present invention which could be packaged or provided as a kit with one or more of sterile package assembly 350, including fluid delivery device 10. There is no need for the remote control device 100 to be sterilized, so if the fluid delivery device 10 was sterilized, one or more sterile package assemblys 350 can be boxed or otherwise packaged with a single remote control device 100 along with one or more other devices.

Fig. 14c depicts a separate data collection assembly 500 of the system of the present invention that is used to gather data relating to a physiologic parameter. The data collection assembly 500 is designed to communicate with either or both the fluid delivery device 10 or the remote control controller 100. The communication may be accomplished with a direct electrical connection or via wireless communication as described above. The data collection assembly may include a DCA sensor assembly 520, not shown. The DCA sensor assembly 520 may include means of quantifying a physiologic sample. For example, the physiologic sample may be blood, and the physiologic parameter to be quantified may be blood glucose. Additionally or alternatively, the data collection assembly 500 may include a DCA sensor communication element 540 that communicates with a sensor which measures of physiologic parameter. An example would include an implanted blood glucose sensor, wherein the DCA sensor communication element comprises a light source, and a means of measuring type, quantity and quality of the reflected light from the implanted sensor. Alternatively, the DCA sensor communication element 540 may work with a separate diagnostic device such as a Glucometer. Other communication means may include infrared or near infrared light, and samples taken may include interstitial fluid.

Fig 14d depicts a therapeutic fluid supply 250, which may consist of a vial of drug such as insulin. The drug, in one or more vials, which has been sterilized and made otherwise biocompatible for use, can be packaged with one or more sterile package assemblies 350 as well as with one or more remote control devices 100. Additional devices may be included in the kit if desired.

Figure 14e depicts a sterile infusion set assembly 407 including the transcutaneous infusion set 400 described hereabove and packaged in an infusion set pouch 406. The infusion set 400 includes an infusion set Luer 401 connected to infusion set flexible tubing 404 and terminating in an infusion set penetrating cannula 405. An optional set of infusion set wings 403 can be included to attach the infusion set 400 to the patient's skin. In the preferred embodiment of fluid delivery device 10, the transcutaneous delivery means are integrated into exit port assembly 70, however in an alternative embodiment, the exit port assembly 70 can be attached to infusion set 400. In this particular embodiment, it may be desirable to kit sterile infusion set assemblies 407 with any quantity of one or more of sterile assembly pack 350, fluid delivery device 10, remote control device 100 or therapeutic fluid supply 250.

The fluid delivery device 10 of the system of the present invention is intended to be low cost and potentially disposable. It may be advantageous for one or more of the components to be biodegradable, since replacement of the device every two to five days has many advantages, it would also generate a fair amount of waste. The fluid delivery device 10 may include a preinstalled battery as its power supply 80. In order to prevent the battery from powering the electronics of fluid delivery device 10 before its intended use, a mechanical switch may be included, connecting the battery contacts to the electronics prior to programming with the remote control device 100. A simplistic version of the switch design may be an insulating material between the battery contacts of power supply 80 and the electrical connection to the electronic microcontroller 50 as is described above in relation to the fluid delivery device 10 embodiment depicted in Fig. 14a. The insulating material could be designed to protrude through housing 20, and be removable by the user, not shown. The user could pull the insulating material and remove it, simultaneously connecting the battery contacts with the electrical connection to the electronic microcontroller.

The fluid delivery device 10 of the present invention may be filled with the therapeutic fluid by the device manufacture, a pharmaceutical company, or another manufacturer prior to its shipment to the hospital, pharmacy or patient. Certain drugs require refrigeration or other special environmental conditions, requiring the prefilled fluid delivery device to be refrigerated or otherwise handled to meet special requirements. Insulin is a drug that requires refrigeration if it is to be stored for a prolonged period of time. Hoechst, of Frankfurt Germany, is developing insulin that is stable at higher temperatures. Drugs that are stable at room temperature, such as the developmental insulin of Hoechst, allow simple filling and handling of the fluid delivery device 10, greatly simplifying the requirements for the patient.

Various methods of using the fluid delivery device 10 are included in the present invention and described above. The method of programming the fluid delivery device 10 with remote control device 100 as well as the attachment and use of the peripheral devices including transcutaneous infusion sets and diagnostic devices such as glucometers are described. The ability of the complete system including fluid delivery device 10, remote control device 100 and data collection assembly 500 to provide a low cost, sophisticated system for therapeutic fluid delivery, wherein data regarding a physiologic parameter is collected is a definitive need. The system can gather the data by including integrated sensor and other means of analyzing a particular physiologic parameter, including measurement of a sample, such as blood or other bodily fluid from the patient. Alternatively, the system can work with a separate diagnostic device which measures the physiologic parameter and communicates with the system via direct electronic connection or wireless communication.

Also relevant to the system, is the ability to update the internal programming of either the fluid delivery device 10 or the remote control device 100 by the corresponding device. If the data collection assembly 500 is a stand alone device, internal programming can also be updated by either the fluid delivery device 10 or remote control device 100, or the data collection assembly 500 could update the fluid delivery device 10 or programming of the remote control device 100.

Methods of filling the fluid delivery device 10 with therapeutic fluid during the manufacturing process as well as by the user have been described. Methods and timing of sterilization and packaging of part or all of the system of the present invention including fluid delivery device 10 and a therapeutic fluid have also been described.

Although exemplary embodiments of the invention have been shown and described, many changes, modifications and substitutions may be made by those having ordinary skill in the art without necessarily departing from the scope of this invention as defined in the claims. For example, the fluid delivery device of this invention is intended to be low cost, light weight, simple to use and potentially disposable by removing a majority of the user interface, including electromechanical switches, from the fluid delivery device, and including a separate controller to replace those functions. A reservoir, fluid dispenser, transcutaneous fluid administration means, solid state electronics and wireless communications are included in the fluid delivery device to perform its intended function. While various means of reservoir construction, pressurization means, fluid pumping means, fluid metering means, transcutaneous delivery, electronic control and wireless communications have been discussed in this application, alternatives to each of these areas can be made without departing from the scope of the invention as defined in the claims. Additionally, while diagnostic devices such as a Glucometer have been referenced in this application, many currently available diagnostic devices may be used and potentially modified to work with the system, especially those diagnostic devices whose output in some way is relevant to volume, timing and other parameters of medicinal fluid delivery.

In addition, where this patent application has listed the steps of a method or procedure in a specific order, it may be possible or even expedient in certain circumstances to change the order in which some steps are performed, and it is intended that the particular steps of the method or procedure claims set forth herebelow not be construed as being order-specific unless such order specificity is expressly stated in the claim.

## Claims

1. System for delivering fluid to a patient, comprising:
A) a fluid delivery device (10) comprising a disposable assembly (94) and a reusable assembly (93), the reusable assembly being attachable to the disposable assembly,
the disposable assembly (94) including:
an exit port assembly (70) including a transcutaneous access tool (72), a fluid metering means (48) for causing fluid from a reservoir (30) to flow to the exit port assembly (70) and a disposable assembly housing (20D) surrounding the fluid metering means (48) and the reservoir 30, and
the reusable assembly (93) including:
a local processor (50) connected to metering control means (46) and programmed to cause fluid flow to the exit port assembly (70) based upon flow instructions to the metering control means (46), which activates the fluid metering means (48), the local processor (50) of the fluid delivery device is programmed to provide flow information, a local communication unit (60) connected to the local processor (50), the local communication unit (60) includes a wireless transmitter for transmitting the flow information from the local processor (50), , and a reusable assembly housing (20R) surrounding the local processor (50), the metering control means (46), and the local communication unit (60), and
wherein the housings (20D, 20R) are free of user output components for providing the flow information from the local processor (50) to a user;
B) a remote control device (100) separate from the fluid delivery device (10) and including, a remote processor, user interface components (110, 120) connected to the remote processor, and a remote communication unit connected to the remote processor and adapted to communicate with the local communication unit (60) of the fluid delivery device (10) such that information can be transferred between the local processor (50) and the remote processor, the remote communication unit of the remote control device includes a remote receiver (130) for receiving the flow information from the local transmitter, and the user interface components of the remote control device include output components connected to the remote processor for allowing a user to receive the flow information; and
C) at least one data collection assembly (500) adapted to measure, monitor, calculate, and/or store a physiologic parameter of a patient, wherein the data collection assembly (500) is integrated into the fluid delivery device (10) in either the disposable assembly (94) or the reusable assembly (93).

2. The system of Claim 1, wherein the data collection assembly (500) includes a sensor assembly (520) that measures the physiologic parameter.

3. The system of Claim 2, wherein the sensor assembly (520) is remotely deployable with respect to the data collection assembly (500), and the data collection assembly communicates with the sensor assembly (520).

4. The system of Claim 3, wherein the data collection assembly (500) also includes a storage element adapted to store the measurements received from the remote sensor.

5. The system claimed in any one of claims 2 to 4, wherein the data collection assembly (500) is in fluid or electrical communication with the sensor assembly (520).

6. The system as claimed in any one of the preceding claims, wherein said system further includes an alarm (103, 223).

7. The system claimed in claim 6, wherein the data collection assembly (500) activates the alarm when a predetermined level of the physiologic parameter is reached.

8. The system claimed in any one of the preceding claims, wherein the fluid delivery device (10) includes a subcutaneous access tool (72) in fluid communication with the data collection assembly (500).

9. The system claimed in any one of the preceding claims, wherein the remote control device (100) comprises a personal data assistant.

10. The system claimed in any one of the preceding claims, wherein the data collection assembly (500) is adapted to be worn on an arm of a patient.

11. The system claimed in any one of the preceding claims, wherein the transcutaneous access tool (72) comprises a needle.

12. The system claimed in any one of the preceding claims, wherein the communication between the remote control device (100) and the fluid delivery device (10) is wireless, such as by radio frequency and/or microwave signals or by infra-red and/or optical signals.

13. The system claimed in any one of the preceding claims, wherein at least one of the local processor (50) and the remote processor are programmed to use information from the data collection assembly (500) to calculate the flow instructions, or to determine an alarm condition, or to determine or monitor a variable of the flow instructions.

14. The system claimed in any one of the preceding claims, wherein the fluid delivery device (10) delivers fluid only upon receiving a signal from the remote control device (100).

15. The system claimed in any one of the preceding claims, wherein the fluid delivery device (10) further comprises projections (204) having adhesive adapted to attach the fluid delivery device to a skin surface of a patient.

16. The system of claim 15, wherein the projections are unitary and extend from the fluid delivery device (10) such that the fluid delivery device is positioned between the unitary projections (204) and the skin surface (210).

17. The system of claim 15 or 16, wherein the exit port assembly (70) of the fluid delivery device (10) includes tubing secured to the skin surface (210) by one of the projections (204).

18. The system claimed in claim 17, wherein one projection (204) includes a transcutaneous penetrating cannula (72) connected to the tubing.

19. The system claimed in any one of claims 15 to 18, wherein the projections (204) extend from opposing sides of the fluid delivery device or from all sides of the fluid delivery device (10).

20. The system claimed in any one of the preceding claims, wherein the reservoir (30) contains a therapeutic fluid such as insulin.

21. The system claimed in any one of the preceding claims, wherein the fluid delivery device (10) further comprises a fill port connected to the reservoir (30).

22. The system claimed in any one of the preceding claims, wherein the reservoir (30) is made of a flexible material and collapses as emptied, wherein the reservoir is preferably pressurized e.g. by a spring pressurizing the reservoir.

23. The system claimed in any one of the preceding claims, wherein: the local processor (50) of the fluid delivery device (10) is programmed to cause a flow of fluid to the exit port assembly (70) based solely on flow instructions from the separate, remote control device (100); the local communication unit (60) includes a wireless receiver for receiving the flow instructions and delivering the flow instructions to the local processor; the remote communication unit of the remote control device includes a remote transmitter for sending the flow instructions to the local receiver; and the user interface components of the remote control device include input components connected to the remote processor for allowing a user to enter the flow instructions.

24. The system of claim 23, wherein the fluid delivery device (10) includes a housing (20) containing the exit port assembly (70), the dispenser (40), the local processor (50), and the wireless receiver, and wherein the housing is free of user input components for providing the flow instructions to the local processor.

25. The system as claimed in claim 24, wherein: the local processor (50) is programmed to receive at least some of the flow instructions from the remote control unit (100); the local communication unit (60) also includes a wireless receiver connected to the local processor; the remote communication unit of the remote control device includes a remote transmitter (130) for sending the flow instructions to the local receiver; and the user interface components (110, 120) of the remote control device include input components connected to the remote processor for allowing a user to enter the flow instructions.

26. The system claimed in any one of the preceding claims, wherein the fluid delivery device (10) is packaged for shipping and handle prior to use in a container, wherein the container and the fluid delivery device are preferably arranged such that opening the container changes the electronic state of the fluid delivery device, and more preferably wherein opening the container connects a power supply (80) of the fluid delivery device to the local processor (50) of the fluid delivery device.

27. The system claimed in any one of the preceding claims, wherein the dispenser (40) includes an expandable accumulator (43A, 44A), an inlet valve (41) controlling flow from a reservoir (30) into the accumulator and an outlet valve (42) controlling flow between the accumulator and the exit port assembly (72) and/or the dispenser includes two expandable accumulators (43B, 44B), and/or the dispenser comprises a pump for pumping fluid from a reservoir to the exit port assembly.

28. The system as claimed in any one of the preceding claims, further including at least one local sensor (220) connected to the local processor (50) and comprising at least one of an occlusion detector, a reservoir volume transducer, a reservoir empty detector, a leak detector, a pressure transducer, a fluid contact detector, an impedance monitor, a voltage detector, a photodetector, and a vibration monitor.

29. The system as claimed in any one of the preceding claims, wherein the local processor (50) includes programming which can be updated by the remote control device (100), and/or the data collection assembly (500) is adapted to communicate with a separate diagnostic device.

30. A kit including system as claimed in any one of the preceding claims, and further comprising a subcutaneous access tool for connection to the exit port assembly (72) of the fluid delivery device (10).

31. A kit according to Claim 30, including a single remote control device (100), a single data collection assembly (500), a plurality of fluid delivery devices (10), and a plurality of subcutaneous access tools.

32. A kit according to Claim 30, wherein each fluid delivery device (10) includes a bar code (26) and the remote control device (100) includes a bar code scanner.

## Patentansprüche

1. System zur Abgabe von Flüssigkeit an einen Patienten, wobei das System Folgendes umfasst:
A) eine Flüssigkeitsabgabevorrichtung (10), die eine Einweganordnung (94) und eine wiederverwendbare Anordnung (93) umfasst; wobei die wiederverwendbare Anordnung an der Einweganordnung angebracht werden kann,
wobei die Einweganordnung (94) Folgendes aufweist:
eine Austrittsanschlussanordnung (70), die ein transkutanes Zugangsinstrument (72) aufweist, eine Flüssigkeitsdosiereinrichtung (48), um zu bewirken, dass Flüssigkeit von einem Reservoir (30) zu der Austrittsanschlussanordnung (70) fließt, und ein Gehäuse (20D) der Einweganordnung, das die Flüssigkeitsdosiereinrichtung (48) und das Reservoir 30 umschließt, und
wobei die wiederverwendbare Anordnung (93) Folgendes aufweist:
einen lokalen Prozessor (50), der an eine Dosiersteuerungseinrichtung (46) angeschlossen ist und dazu programmiert ist, einen Flüssigkeitsfluss zu der Austrittsanschlussanordnung (70) auf Basis von Flussanweisungen an die Dosiersteuerungseinrichtung (46) zu bewirken, der die Flüssigkeitsdosiereinrichtung (48) aktiviert, wobei der lokale Prozessor (50) der Flüssigkeitsabgabevorrichtung dazu programmiert ist, Flussinformationen bereitzustellen, eine lokale Kommunikationseinheit (60), die an den lokalen Prozessor (50) angeschlossen ist, wobei die lokale Kommunikationseinheit (60) einen drahtlosen Sender zum Senden der Flussinformationen von dem lokalen Prozessor (50) aufweist, und ein Gehäuse (20R) der wiederverwendbaren Anordnung, das den lokalen Prozessor (50), die Dosiersteuerungseinrichtung (46) und die lokale Kommunikationseinheit (60) umschließt; und
wobei die Gehäuse (20D, 20R) frei von Benutzerausgabekomponenten zum Bereitstellen der Flussinformationen von dem lokalen Prozessor (50) an einen Benutzer sind;
B) eine Fernsteuerungsvorrichtung (100), die von der Flüssigkeitsabgabevorrichtung (10) getrennt ist und einen dezentralen Prozessor, Benutzeroberflächenkomponenten (110, 120), die an den dezentralen Prozessor angeschlossen sind, und eine dezentrale Kommunikationseinheit aufweist, die an den dezentralen Prozessor angeschlossen ist und dazu eingerichtet ist, mit der lokalen Kommunikationseinheit (60) der Flüssigkeitsabgabevorrichtung (10) zu kommunizieren, so dass Informationen zwischen dem lokalen Prozessor (50) und dem dezentralen Prozessor übertragen werden können, wobei die dezentrale Kommunikationseinheit der Fernsteuerungsvorrichtung einen dezentralen Empfänger (130) zum Empfangen der Flussinformationen von dem lokalen Sender aufweist und die Benutzeroberflächenkomponenten der Fernsteuerungsvorrichtung Ausgabekomponenten aufweisen, die mit dem dezentralen Prozessor verbunden sind, um einem Benutzer zu ermöglichen, die Flussinformationen zu empfangen; und
C) mindestens eine Datenerfassungsanordnung (500), die dazu eingerichtet ist, einen physiologischen Parameter eines Patienten zu messen, zu überwachen, zu berechnen und/oder zu speichern, wobei die Datenerfassungsanordnung (500) in die Flüssigkeitsabgabevorrichtung (10) in entweder der Einweganordnung (94) oder der wiederverwendbaren Anordnung (93) integriert ist.

2. System nach Anspruch 1, wobei die Datenerfassungsanordnung (500) eine Sensoranordnung (520) aufweist, die den physiologischen Parameter misst.

3. System nach Anspruch 2, wobei die Sensoranordnung (520) in Bezug auf die Datenerfassungsanordnung (500) aus der Ferne eingesetzt werden kann und die Datenerfassungsanordnung mit der Sensoranordnung (520) kommuniziert.

4. System nach Anspruch 3, wobei die Datenerfassungsanordnung (500) außerdem ein Speicherelement aufweist, das dazu eingerichtet ist, die von dem dezentralen Sensor empfangenen Messungen zu speichern.

5. System nach einem der Ansprüche 2 bis 4, wobei die Datenerfassungsanordnung (500) in Flüssigkeits- oder elektrischer Verbindung mit der Sensoranordnung (520) steht.

6. System nach einem der vorhergehenden Ansprüche, wobei das System weiterhin einen Alarm (103, 223) aufweist.

7. System nach Anspruch 6, wobei die Datenerfassungsanordnung (500) den Alarm aktiviert, wenn ein vorherbestimmtes Niveau des physiologischen Parameters erreicht wird.

8. System nach einem der vorhergehenden Ansprüche, wobei die Flüssigkeitsabgabevorrichtung (10) ein subkutanes Zugangsinstrument (72) in Flüssigkeitsverbindung mit der Datenerfassungsvorrichtung (500) aufweist.

9. System nach einem der vorhergehenden Ansprüche, wobei die Fernsteuerungsvorrichtung (100) einen Minicomputer (PDA) umfasst.

10. System nach einem der vorhergehenden Ansprüche, wobei die Datenerfassungsanordnung (500) dazu eingerichtet ist, an einem Arm eines Patienten getragen zu werden.

11. System nach einem der vorhergehenden Ansprüche, wobei das transkutane Zugangsinstrument (72) eine Nadel umfasst.

12. System nach einem der vorhergehenden Ansprüche, wobei die Kommunikation zwischen der Fernsteuerungsvorrichtung (100) und der Flüssigkeitsabgabevorrichtung (10) drahtlos ist, wie durch Hochfrequenz- und/oder Mikrowellensignale oder durch Infrarot- und/oder optische Signale.

13. System nach einem der vorhergehenden Ansprüche, wobei der lokale Prozessor (50) und/oder der dezentrale Prozessor dazu programmiert sind, Informationen von der Datenerfassungsanordnung (500) dazu zu verwenden, die Flussanweisungen zu berechnen oder einen Alarmzustand zu bestimmen oder eine Variable der Flussanweisungen zu bestimmen oder zu überwachen.

14. System nach einem der vorhergehenden Ansprüche, wobei die Flüssigkeitsabgabevorrichtung (10) Flüssigkeit nur bei Empfang eines Signals von der Fernsteuerungsvorrichtung (100) abgibt.

15. System nach einem der vorhergehenden Ansprüche, wobei die Flüssigkeitsabgabevorrichtung (10) weiterhin Vorsprünge (204) mit Klebstoff umfassen, die dazu eingerichtet sind, die Flüssigkeitsabgabevorrichtung an einer Hautfläche eines Patienten anzubringen.

16. System nach Anspruch 15, wobei die Vorsprünge einheitlich sind und sich derart von der Flüssigkeitsabgabevorrichtung (10) erstrecken, dass die Flüssigkeitsabgabevorrichtung zwischen den einheitlichen Vorsprüngen (204) und der Hautfläche (210) positioniert wird.

17. System nach Anspruch 15 oder 16, wobei die Austrittsanschlussanordnung (70) der Flüssigkeitsabgabevorrichtung (10) einen Schlauch aufweist, der durch einen der Vorsprünge (204) an der Hautfläche (210) befestigt werden.

18. System nach Anspruch 17, wobei ein Vorsprung (204) eine transkutane Durchstechkanüle (72) aufweist, die mit dem Schlauch verbunden ist.

19. System nach einem der Ansprüche 15 bis 18, wobei die Vorsprünge (204) sich von entgegengesetzten Seiten der Flüssigkeitsabgabevorrichtung oder von allen Seiten der Flüssigkeitsabgabevorrichtung (10) erstrecken.

20. System nach einem der vorhergehenden Ansprüche, wobei das Reservoir (30) eine therapeutische Flüssigkeit wie Insulin enthält.

21. System nach einem der vorhergehenden Ansprüche, wobei die Flüssigkeitsabgabevorrichtung (10) weiterhin einen Einfüllanschluss umfasst, der mit dem Reservoir (30) verbunden ist.

22. System nach einem der vorhergehenden Ansprüche, wobei das Reservoir (30) aus einem flexiblen Material hergestellt ist und beim Leeren zusammenfällt, wobei das Reservoir vorzugsweise unter Druck gesetzt wird, z. B. von einer Feder, die das Reservoir unter Druck setzt.

23. System nach einem der vorhergehenden Ansprüche, wobei: der lokale Prozessor (50) der Flüssigkeitsabgabevorrichtung (10) dazu programmiert ist, einen Flüssigkeitsfluss zu der Austrittsanschlussanordnung (70) ausschließlich auf Basis von Flussanweisungen von der getrennten Fernsteuerungsvorrichtung (100) zu bewirken; die lokale Kommunikationseinheit (60) einen drahtlosen Empfänger zum Empfangen der Flussinformationen und zum Liefern der Flussanweisungen an den lokalen Prozessor aufweist; die dezentrale Kommunikationseinheit der Fernsteuerungsvorrichtung einen dezentralen Sender zum Senden der Flussanweisungen an den lokalen Empfänger aufweist und die Benutzeroberflächenkomponenten der Fernsteuerungsvorrichtung Eingabekomponenten aufweisen, die mit dem dezentralen Prozessor verbunden sind, um einem Benutzer zu ermöglichen, die Flussanweisungen einzugeben.

24. System nach Anspruch 23, wobei die Flüssigkeitsabgabevorrichtung (10) ein Gehäuse (20) aufweist, das die Austrittsanschlussanordnung (70), den Spender (40), den lokalen Prozessor (50) und den drahtlosen Empfänger enthält und wobei das Gehäuse frei von Benutzereingabekomponenten zum Bereitstellen der Flussanweisungen an den lokalen Prozessor sind.

25. System nach Anspruch 24, wobei: der lokale Prozessor (50) dazu programmiert ist, mindestens einige der Flussanweisungen von der Fernsteuerungseinheit (100) zu empfangen; die lokale Kommunikationseinheit (60) außerdem einen drahtlosen Empfänger aufweist, der mit dem lokalen Prozessor verbunden ist; die dezentrale Kommunikationseinheit der Fernsteuerungsvorrichtung einen dezentralen Sender (130) zum Senden der Flussanweisungen an den lokalen Empfänger aufweist und die Benutzeroberflächenkomponenten (110, 120) der Fernsteuerungsvorrichtung Eingabekomponenten aufweisen, die mit dem dezentralen Prozessor verbunden sind, um einem Benutzer zu ermöglichen, die Flussanweisungen einzugeben.

26. System nach einem der vorhergehenden Ansprüche, wobei die Flüssigkeitsabgabevorrichtung (10) zum Versand und zur Handhabung vor Gebrauch in einen Behälter verpackt wird, wobei der Behälter und die Flüssigkeitsabgabevorrichtung vorzugsweise derart angeordnet werden, dass das Öffnen des Behälters den elektronischen Zustand der Flüssigkeitsabgabevorrichtung ändert und mehr bevorzugt wobei das Öffnen des Behälters eine Energieversorgung (80) der Flüssigkeitsabgabevorrichtung mit dem lokalen Prozessor (50) der Flüssigkeitsabgabevorrichtung verbindet.

27. System nach einem der vorhergehenden Ansprüche, wobei der Spender (40) einen erweiterbaren Speicher (43A, 44A), ein Einlassventil (41), das den Fluss von einem Reservoir (30) in den Speicher steuert, und ein Auslassventil (42), das den Fluss zwischen dem Speicher und der Austrittsanschlussanordnung (72) steuert, aufweist und/oder der Spender zwei erweiterbare Speicher (43B, 44B) aufweist und/oder der Spender eine Pumpe zum Pumpen von Flüssigkeit von einem Reservoir zu der Austrittsanschlussanordnung umfasst.

28. System nach einem der vorhergehenden Ansprüche, das weiterhin mindestens einen lokalen Sensor (220) aufweist, der mit dem lokalen Prozessor (50) verbunden ist und einen Verstopfungsdetektor, einen Reservoirvolumengeber, einen Reservoir-Leer-Detektor, einen Leckdetektor, einen Druckwandler, einen Flüssigkeitskontaktdetektor, eine Impedanzüberwachungsvorrichtung, einen Spannungsprüfer, einen Fotodetektor und eine Vibrationsüberwachungsvorrichtung umfasst.

29. System nach einem der vorhergehenden Ansprüche, wobei der lokale Prozessor (50) eine Programmierung aufweist, die von der Fernsteuerungsvorrichtung (100) aktualisiert werden kann, und/oder die Datenerfassungsanordnung (500) dazu eingerichtet ist, mit einer getrennten Diagnosevorrichtung zu kommunizieren.

30. Kit, das ein System nach einem der vorhergehenden Ansprüche aufweist und weiterhin ein subkutanes Zugangsinstrument zur Verbindung mit der Austrittsanschlussanordnung (72) der Flüssigkeitsabgabevorrichtung (10) umfasst.

31. Kit nach Anspruch 30, das eine einzige Fernsteuerungsvorrichtung (100), eine einzige Datenerfassungsanordnung (500), mehrere Flüssigkeitsabgabevorrichtungen (10) und mehrere subkutane Zugangsinstrumente aufweist.

32. Kit nach Anspruch 30, wobei jede Flüssigkeitsabgabevorrichtung (10) einen Strichcode (26) aufweist und die Fernsteuerungsvorrichtung (100) einen Strichcodeleser aufweist.

## Revendications

1. Une méthode d'administration d'un fluide à un patient, comprenant
A) un dispositif d'administration du fluide (10) composé d'un ensemble jetable (94) et d'un ensemble réutilisable (93), l'ensemble réutilisable pouvant être fixé à l'ensemble jetable, et
l'ensemble jetable (94), étant composé :
d'un orifice de sortie (70) comprenant un instrument d'accès transcutané (72), un doseur de fluide (48) déterminant l'écoulement d'un fluide d'un réservoir (30) à un orifice de sortie (70), et d'un boîtier jetable (20D) entourant le doseur de fluide (48) et le réservoir (30),
l'ensemble réutilisable (93), étant composé :
d'un processeur local (50) relié à un régulateur de dosage (46) et programmé pour assurer l'écoulement d'un fluide à l'orifice de sortie (70), conformément à des instructions d'écoulement émises au régulateur de dosage (46), qui déclenche le doseur de fluide (48), le processeur local (50) du dispositif d'administration du fluide étant programmé pour fournir des informations sur le débit, un dispositif de communication local (60) relié au processeur local (50), le dispositif de communication local (60) comprenant un transmetteur sans fil assurant la transmission d'informations sur l'écoulement provenant du processeur local (50), et un boîtier réutilisable (20R) renfermant le processeur local (50), le régulateur de dosage (46), et le dispositif de communication local (60), et
au sein duquel les boîtiers (20D, 20R) sont dénués de composants de sortie par l'utilisateur assurant la fourniture d'informations sur le débit du processeur local (50) a un utilisateur ;
B) un dispositif de télécommande (100) séparé du dispositif d'administration du fluide (10), comprenant un processeur éloigné, des composants d'interface avec l'utilisateur (110, 120) raccordés au processeur éloigné, et un dispositif de communications éloigné raccordé au processeur éloigné et adapté pour communiquer avec le dispositif de communication local (60) du dispositif d'administration du fluide (10), de telle façon que ces informations puissent être transférées entre le processeur local (50) et le processeur éloigné, le dispositif de communications éloigné du dispositif de télécommande comprenant un récepteur à distance (130) recevant les informations sur le débit du transmetteur local, et les composants d'interface avec l'utilisateur du dispositif de télécommande comprenant des composants de sortie reliés au processeur éloigné, en permettant à l'utilisateur de recevoir les informations sur le débit ; et
C) au minimum un ensemble de collecte de données (500) adapté pour mesurer, contrôler, calculer et/ou stocker un paramètre physiologique d'un patient, dans lequel l'ensemble de collecte de données (500) est intégré au dispositif d'administration du fluide (10) soit dans l'ensemble jetable (94) soit dans l'ensemble réutilisable (93).

2. Le système selon la revendication 1, dans lequel l'ensemble de collecte de données (500) comprend un détecteur (520) mesurant le paramètre physiologique.

3. Le système selon la revendication 2, dans lequel le détecteur (520) peut être déployé à distance par rapport à l'ensemble de collecte de données (500), et l'ensemble de collecte de données communique avec le détecteur (520).

4. Le système selon la revendication 3, dans lequel l'ensemble de collecte de données (500) comprend également un élément de stockage adapté pour mémoriser les relevés effectués par le détecteur éloigné.

5. Le système selon une quelconque des revendications 2 à 4, dans lequel l'ensemble de collecte de données (500) est en communication fluide ou électrique avec le détecteur (520).

6. Le système selon une quelconque des revendications précédentes, dans lequel ledit système comprend également une alarme (103, 223).

7. Le système selon la revendication 6, dans lequel l'ensemble de collecte de données (500) déclenche une alarme lorsqu'un niveau prédéterminé du paramètre physiologique est atteint.

8. Le système selon une quelconque des revendications précédentes, dans lequel le dispositif d'administration du fluide (10) comprend un instrument d'accès sous-cutané (72) en communication fluide avec l'ensemble de collecte de données (500).

9. Le système selon une quelconque des revendications précédentes, dans lequel le dispositif de télécommande (100) comprend un assistant numérique personnel.

10. Le système selon une quelconque des revendications précédentes, dans lequel l'ensemble de collecte de données (500) est adapté pour être porté sur le bras d'un patient.

11. Le système selon une quelconque des revendications précédentes, dans lequel l'instrument d'accès sous-cutané (72) comprend une aiguille.

12. Le système selon une quelconque des revendications précédentes, dans lequel les communications entre le dispositif de télécommande (100) et le dispositif d'administration du fluide (10) s'effectuent sans fil, par exemple par des signaux en radiofréquence et/ou à micro-ondes ou par signaux infrarouges et/ou optiques.

13. Le système selon une quelconque des revendications précédentes, dans lequel au moins un des processeurs, que sont le processeur local (50) et le processeur éloigné, est programmé pour utiliser des informations provenant de l'ensemble de collecte de données (500) pour calculer les instructions relatives au débit, ou déterminer le déclenchement d'une alarme, ou encore pour déterminer ou contrôler une variable des instructions pour le débit.

14. Le système selon une quelconque des revendications précédentes, dans lequel le dispositif d'administration du fluide (10) n'assure l'administration de fluide que lorsqu'il reçoit un signal du dispositif de télécommande (100).

15. Le système selon une quelconque des revendications précédentes, dans lequel le dispositif d'administration du fluide (10) comprend également des saillies (204) comprenant un adhésif adapté pour la fixation du dispositif d'administration du fluide sur la peau d'un patient.

16. Le système selon la revendication 15, dans lequel les saillies sont unitaires, et s'étendent du dispositif d'administration du fluide (10) de sorte que le dispositif d'administration du fluide se place entre les saillies unitaires (204) et la surface de la peau (210).

17. Le système selon la revendication 15 ou 16, dans lequel l'orifice de sortie (70) du dispositif d'administration du fluide (10) comprend un tube fixé sur la surface de la peau (210) par une des saillies (204).

18. Le système selon la revendication 17, dans lequel une saillie (204) comprend une canule à pénétration transcutanée (72) reliée au tube.

19. Le système selon une quelconque des revendications 15 à 18, dans lequel les saillies (204) s'étendent de côtés opposés du distributeur de fluide, ou de tous les côtés du dispositif d'administration du fluide (10).

20. Le système selon une quelconque des revendications précédentes, dans lequel le réservoir (30) contient un fluide thérapeutique, par exemple de l'insuline.

21. Le système selon une quelconque des revendications précédentes, dans lequel le dispositif d'administration du fluide (10) comprend un orifice de remplissage relié au réservoir (30).

22. Le système selon une quelconque des revendications précédentes, dans lequel le réservoir (30) est réalisé avec une matière flexible et s'affaisse au fur et à mesure qu'il se vide, et est de préférence maintenu sous pression, p. ex. par un ressort maintenant le réservoir sous pression.

23. Le système selon une quelconque des revendications précédentes, dans lequel : le processeur local (50) du dispositif d'administration du fluide (10) est programmé pour déterminer le refoulement d'un fluide à l'orifice de sortie (70) en réponse exclusivement à des instructions d'écoulement provenant du dispositif de télécommande (100) séparé ; le dispositif de communication local (60) comprend un transmetteur sans fil assurant la réception d'instructions sur le débit et transmettant des instructions sur le débit au processeur local ; le dispositif de communications éloigné du dispositif de télécommande comprend un transmetteur éloigné assurant la transmission d'instructions sur le débit au récepteur local ; et les composants de l'interface utilisateur du dispositif de télécommande comprennent des composants reliés au processeur éloigné, en permettant à un utilisateur d'entrer des instructions sur le débit.

24. Le système selon la revendication 23, dans lequel le dispositif d'administration du fluide (10) comprend un boîtier (20) contenant l'orifice de sortie (70), le distributeur (40), le processeur local (50), et le transmetteur sans fil, et dans lequel le boîtier est dénué de composants d'entrée par l'utilisateur pour la fourniture d'instructions sur le débit au processeur local.

25. Le système selon la revendication 24, dans lequel le processeur local (50) est programmé pour recevoir au minimum certaines des instructions sur le débit provenant du dispositif de télécommande (100) ; le dispositif de communication local (60) comprend également un récepteur sans fil relié au processeur local ; le dispositif de communications éloigné du dispositif de télécommande comprend un transmetteur éloigné (130) transmettant les instructions sur le débit au récepteur local ; et les composants d'interface avec l'utilisateur (110, 120) du dispositif de communications éloigné comprend des composants d'entrée reliés au processeur éloigné pour permettre à l'utilisateur d'entrer les instructions sur le débit.

26. Le système selon une quelconque des revendications précédentes, dans lequel le dispositif d'administration du fluide (10) est conditionné pour le transport et la manipulation, préalablement à l'emploi, dans un conteneur, dans lequel le conteneur et le dispositif d'administration du fluide sont disposés, de préférence, de sorte que l'ouverture du conteneur modifie l'état électronique du dispositif d'administration du fluide, et, de préférence, dans lequel l'ouverture du conteneur relie une alimentation électrique (80) du dispositif d'administration du fluide au processeur local (50) du dispositif d'administration du fluide.

27. Le système selon une quelconque des revendications précédentes, dans lequel le distributeur (40) comprend un accumulateur expansible (43A, 44A), un robinet d'entrée (41) assurant la régulation du débit, provenant d'un réservoir (30), dans l'accumulateur, et un robinet de sortie (42) assurant la régulation du débit entre l'accumulateur et l'orifice de sortie (72) et/ou le distributeur comprend deux accumulateurs expansibles (43B, 44B), et/ou le distributeur comprend une pompe assurant le pompage d'un fluide d'un réservoir à l'ensemble de l'orifice de sortie.

28. Le système selon une quelconque des revendications précédentes, comprenant également au moins un détecteur local (220) relié au processeur local (50), et comprenant au minimum un détecteur d'occlusion, un transducteur de volume du réservoir, un détecteur de réservoir vide, un détecteur de fuite, un transducteur de pression, un détecteur de contact du fluide, un contrôleur à impédance, un détecteur de tension, une cellule photoélectrique, et un contrôleur de vibrations.

29. Le système selon une quelconque des revendications précédentes, dans lequel le processeur local (50) comprend une programmation pouvant être actualisée par le dispositif de télécommande (100), et/ou l'ensemble de collecte de données (500) est adapté pour communiquer avec un dispositif de diagnostic séparé.

30. Un kit incorporant le système revendiqué dans une quelconque des revendications précédentes, et comprenant également un outil d'accès sous-cutané pour le raccordement à l'orifice de sortie (72) du dispositif d'administration du fluide (10).

31. Un kit selon la revendication 30, comprenant un dispositif de télécommande (100), un ensemble de collecte de données (500) unique, une série de dispositifs d'administration de fluides (10), et une série d'instruments d'accès sous-cutané.

32. Un kit selon la revendication 30, dans lequel chaque dispositif d'administration du fluide (10) comprend un code à barres (26), et le dispositif de télécommande (100) comprend un scanner pour code à barres.
